# EUROPEAN PATENT APPLICATION

(11) **EP 4 541 555 A1**
(43) Date of publication of application: **23.04.2025**
(21) Application number: 23835524.2
(22) Date of filing: 04.07.2023
(51) Int. Cl.: B29C 55/06, C23F 3/00, D04H 1/555, B32B 37/00, A61F 13/15, B29C 65/08

(54) **DEVICE FOR PRODUCING STRETCHABLE LAMINATED SHEET, METHOD FOR PRODUCING STRETCHABLE LAMINATED SHEET, AND WEARABLE ARTICLE USING STRETCHABLE LAMINATED SHEET**

(30) Priority: 07.07.2022 JP 2022109869; 28.12.2022 JP 2022212496
(71) Applicant: Zuiko Corporation, Ibaraki-shi Osaka 5670082 (JP)
(72) Inventor: KOSHIJIMA, Miwa, Ibaraki-shi, Osaka 567-0082 (JP)
(74) Representative: Hasegawa, Kan
(86) International application number: PCT/JP2023/024738
(87) International publication number: WO 2024/009992

(57) **Abstract**

Providing an apparatus for and a method of manufacturing a stretchable laminated sheet that has improved breathability. A stretchable laminated sheet (L) manufacturing apparatus (100) is an apparatus for manufacturing a stretchable laminated sheet in which nonwoven fabric sheets (S1, S2) and a stretchable film (F) are disposed on top of each other and in which the nonwoven fabric sheets and the film are joined together. The stretchable laminated sheet manufacturing apparatus includes a laminating and joining mechanism (48) and a laminated sheet stretching mechanism (60). The laminating and joining mechanism has an extension roll (40) and a horn (43). The laminating and joining mechanism sandwiches between the extension roll and the horn a laminate body (B) in which the nonwoven fabric sheets and the film that is in an extended state are superimposed and heat seals the nonwoven fabric sheets and the film to form the stretchable laminated sheet. The laminated sheet stretching mechanism has a pair of gear rolls (62, 64) having recesses and projections in their peripheral surfaces. The laminated sheet stretching mechanism stretches the stretchable laminated sheet which is conveyed between the pair of gear rolls.

## Description

### TECHNICAL FIELD

The present invention relates to an apparatus for and a method of manufacturing a stretchable laminated sheet used in a wearable article and to a wearable article using the stretchable laminated sheet.

### BACKGROUND ART

Conventionally, apparatus for and methods of manufacturing a stretchable laminated sheet, in which nonwoven fabric sheets and a stretchable film are superimposed and which is used in wearable articles, have been known (e.g., see Patent Document 1 (International Publication No. 2019/150801)). The stretchable laminated sheet is, for example, used for waist portions of the wearable articles and is worn on the waist of the wearer.

Of the sheets used to manufacture the stretchable laminated sheet, the nonwoven fabric sheets are breathable, but the stretchable film is not. For that reason, when the stretchable laminated sheet is used in a wearable article, the problem may arise that the wearer feels stuffiness when wearing the wearable article.

However, in a case where the nonwoven fabric sheets and the film are heat fused in the process of manufacturing the stretchable laminated sheet, the film breaks around the heat fused areas. For that reason, when the wearer wears a wearable article that uses the stretchable laminated sheet, air can pass through the broken portions of the film, so the problem of the wearer feeling stuffiness when wearing the wearable article can be suppressed to some extent.

### CITATION LIST

### Patent Literature

Patent Document 1: International Publication No. 2019/150801

### SUMMARY OF INVENTION

### Technical Problem

However, such breaks in the film around the heat fused areas may not be able to ensure openings with sufficient areas and sufficiently suppress the problem of the wearer feeling stuffiness when wearing the wearable article.

It is an object of the present invention to provide an apparatus for and a method of manufacturing a stretchable laminated sheet that has improved breathability and a wearable article using the stretchable laminated sheet that has improved breathability.

### Solution to Problem

The stretchable laminated sheet manufacturing apparatus of the present invention is an apparatus for manufacturing a stretchable laminated sheet in which a substrate sheet and a stretchable film are disposed on top of each other and in which the substrate sheet and the film are joined together. The stretchable laminated sheet manufacturing apparatus comprises a joining unit and a stretcher. The joining unit has a first joining member and a second joining member. The joining unit is configured to sandwich between the first joining member and the second joining member a laminate body in which the substrate sheet and the film that is in an extended state are superimposed and heat seal the substrate sheet and the film to form the stretchable laminated sheet. The stretcher has a pair of gear rolls having recesses and projections in their peripheral surfaces. The stretcher is configured to stretch the stretchable laminated sheet which is conveyed between the pair of gear rolls.

The stretchable laminated sheet manufacturing method of the present invention is a method of manufacturing a stretchable laminated sheet in which a substrate sheet and a stretchable film are disposed on top of each other and in which the substrate sheet and the film are joined together. The stretchable laminated sheet manufacturing method comprising a sealing step and a stretching step. In the sealing step, the substrate sheet and the film that is in an extended state are heat sealed to form the stretchable laminated sheet. In the stretching step, the stretchable laminated sheet which is conveyed between a pair of gear rolls having recesses and projections in their peripheral surfaces is stretched by the pair of gear rolls.

### Advantageous Effects of Invention

The apparatus for and method of manufacturing a stretchable laminated sheet of the present invention can, by heat sealing the substrate sheet and the film to form the stretchable laminated sheet and then performing stretching with the gear rolls having recesses and projections in their peripheral surfaces, form an opening with a sufficient area in and/or around the place where the substrate sheet and the film have been heat sealed and manufacture the stretchable laminated sheet that has improved breathability.

### BRIEF DESCRIPTION OF DRAWINGS

FIG. 1 is a schematic diagram showing the overall configuration of a stretchable laminated sheet manufacturing apparatus of a first embodiment.
FIG. 2 is a schematic cross-sectional view of the stretchable laminated sheet in which a film is in an extended state.
FIG. 3 is a schematic cross-sectional view of the stretchable laminated sheet in which the film is in a nonextended state.
FIG. 4 is a schematic view of a wearable article that uses the stretchable laminated sheet.
FIG. 5 is a control block diagram of the stretchable laminated sheet manufacturing apparatus of FIG. 1.
FIG. 6 is a schematic enlarged view of an extension roll and a horn of the stretchable laminated sheet manufacturing apparatus of FIG. 1.
FIG. 7 is a drawing showing an example of the arrangement of connected portions formed in the stretchable laminated sheet.
FIG. 8 is a schematic partially enlarged plan of the stretchable laminated sheet before gear stretching and a schematic cross-sectional view taken from the perspective of X1-X1 in the schematic partially enlarged plan view.
FIG. 9 is a schematic partially enlarged plan of the stretchable laminated sheet after the gear stretching and a schematic cross-sectional view taken from the perspective of X2-X2 in the schematic partially enlarged plan view.
FIG. 10 is a photograph of the stretchable laminated sheet before the gear stretching.
FIG. 11 is a photograph of the stretchable laminated sheet after the gear stretching.
FIG. 12 is a schematic diagram showing the configuration of a stretchable laminated sheet manufacturing apparatus of a second embodiment, with illustration of a film former being omitted.
FIG. 13 is a control block diagram of the stretchable laminated sheet manufacturing apparatus of FIG. 12.
FIG. 14 is a schematic diagram showing the configuration of a stretchable laminated sheet manufacturing apparatus of example modification 2, with illustration of the film former being omitted.
FIG. 15 is a control block diagram of the stretchable laminated sheet manufacturing apparatus of FIG. 14.
FIG. 16 is a drawing showing an example of places where the stretchable laminated sheet is used in a wearable article pertaining to one example.
FIG. 17 is a drawing showing an example of places where the stretchable laminated sheet is used in a wearable article pertaining to another example.

### DESCRIPTION OF EMBODIMENTS

Apparatus for and methods of manufacturing a stretchable laminated sheet of embodiments of the present invention will be described below with reference to the drawings.

### <First Embodiment>

### (1) Overview

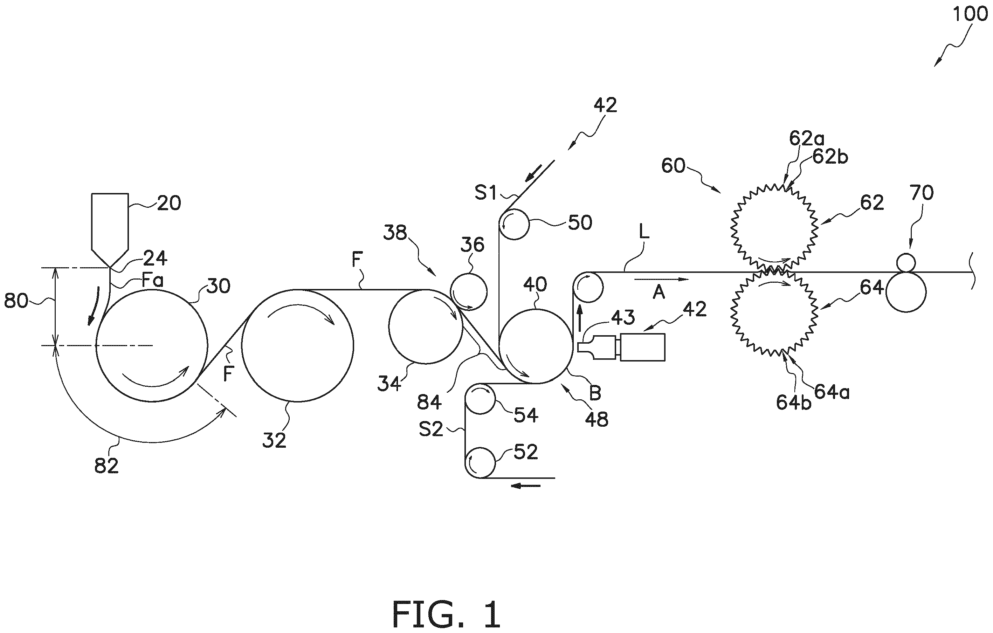

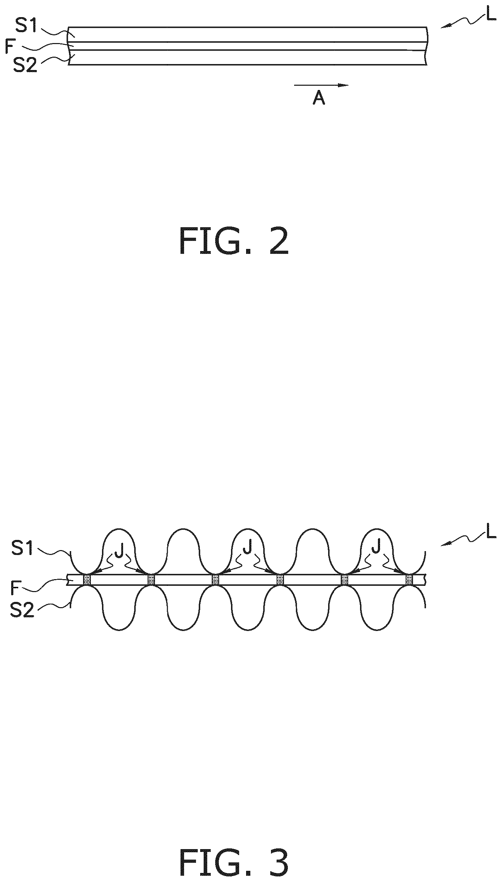

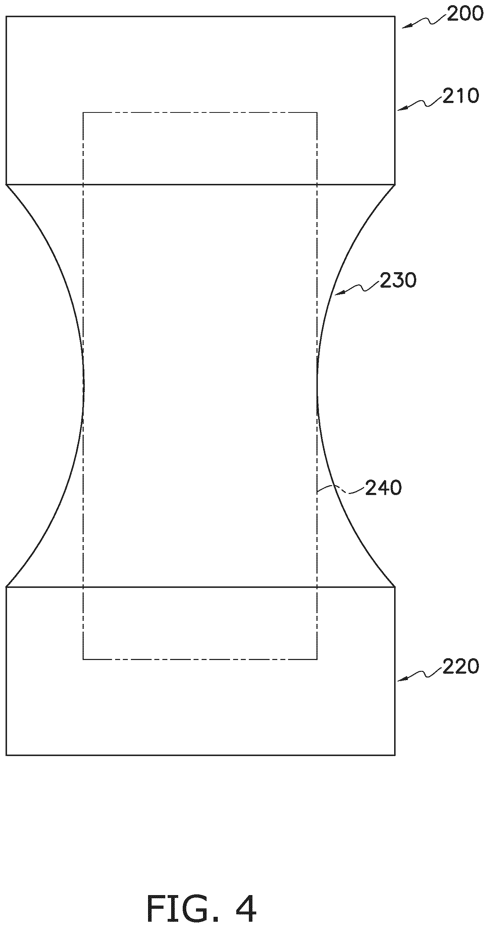

A manufacturing apparatus 100 of stretchable laminated sheet L pertaining to a first embodiment will now be described with reference to FIG. 1 to FIG. 4. FIG. 1 is a schematic diagram showing the overall configuration of the manufacturing apparatus 100 of the stretchable laminated sheet L. FIG. 2 is a cross-sectional view of the stretchable laminated sheet L in which a film F configuring the stretchable laminated sheet L is in an extended state. FIG. 3 is a cross-sectional view of the stretchable laminated sheet L in which the film F is in a nonextended state. FIG. 4 is a schematic view of a wearable article 200 that uses the stretchable laminated sheet L.

### (1-1) Stretchable Laminated Sheet

First, the stretchable laminated sheet L manufactured by the manufacturing apparatus 100 will be described.

The stretchable laminated sheet L is a stretchable sheet formed by laminating multiple sheets/films. The stretchable laminated sheet L, as shown in FIG. 2 and FIG. 3, includes an elastic (stretchable) film F, a first nonwoven fabric sheet S1 serving as an example of a substrate sheet, and a second nonwoven fabric sheet S2 serving as an example of a substrate sheet. In the stretchable laminated sheet L, the film F is disposed between the first nonwoven fabric sheet S1 and the second nonwoven fabric sheet S2. In other words, in the stretchable laminated sheet L, the nonwoven fabric sheets and the film F are disposed on top of each other. In the stretchable laminated sheet L, the first nonwoven fabric sheet S1 and the film F are intermittently joined together at joints J, and the second nonwoven fabric sheet S2 and the film F are intermittently joined together at joints J.

It will be noted that although a three-layer structure comprising the first nonwoven fabric sheet S1, the second nonwoven fabric sheet S2, and the film F is described as an example here, the stretchable laminated sheet may be formed by laminating four or more sheets/films. Furthermore, the stretchable laminated sheet may have a two-layer structure comprising the film F and one of the first nonwoven fabric sheet S1 and the second nonwoven fabric sheet S2.

Moreover, the nonwoven fabric sheets are examples of substrate sheets, but the substrate sheets may be sheets other than nonwoven fabric sheets.

When the stretchable laminated sheet L is being sufficiently extended along a conveyance direction A of the stretchable laminated sheet L in the manufacturing apparatus 100 described later, the first nonwoven fabric sheet S1, the film F, and the second nonwoven fabric sheet S2 are parallel to each other as in FIG. 2. In other words, when the film F of the stretchable laminated sheet L is being extended along the conveyance direction A, the first nonwoven fabric sheet S1, the film F, and the second nonwoven fabric sheet S2 are parallel to each other as in FIG. 2.

When the stretchable laminated sheet L is released from its extended state (in a state in which no force is acting on the stretchable laminated sheet L in a direction along the conveyance direction A), the film F contracts relative to the state in FIG. 2, so the distance between the joints J (the dotted portions) becomes shorter as in FIG. 3. As a result, the first nonwoven fabric sheet S1 and the second nonwoven fabric sheet S2 curve, and crests and troughs are formed in the surface of the stretchable laminated sheet L (see FIG. 3).

The stretchable laminated sheet L is used in wearable articles. Specifically, sheet pieces cut out along a direction orthogonal to the conveyance direction A from the stretchable laminated sheet L are used in wearable articles. It will be noted that, here, the sheet pieces cut out from the stretchable laminated sheet L will also simply be called "the stretchable laminated sheet L." The wearable articles are, for example, disposable diapers or disposable medical gowns, though this is not intended to limit the types of wearable articles in which the stretchable laminated sheet L is used. Furthermore, the stretchable laminated sheet L is widely applicable to, for example, waist members of wearable articles that are worn around the waist of the wearer, wrist cuff members of wearable articles that are worn around the wrists of the wearer, and ankle cuff members of wearable articles that are worn on the ankles of the wearer, though this is not intended to limit the applications of the stretchable laminated sheet L.

As an example, a disposable diaper serving as a wearable article 200 in which the stretchable laminated sheet L is used now will be described with reference to FIG. 4.

As shown in FIG. 4, the wearable article 200 includes a front waist portion 210, a back waist portion 220, a crotch portion 230, and an absorbent article 240. **In** the wearable article 200, the front waist portion 210 and the back waist portion 220 are spaced apart from each other. When the wearable article 200 is worn, the front waist portion 210 and the back waist portion 220 face each other. The crotch portion 230 is connected to the front waist portion 210 and the back waist portion 220. The front waist portion 210 and the back waist portion 220 are interconnected via the crotch portion 230. The absorbent article 240 is disposed so as to straddle the crotch portion 230. When the wearable article 200 is worn, the wearable article 200 is bent at the crotch portion 230, the front waist portion 210 is disposed on the ventral side, and the back waist portion 220 is disposed on the dorsal side. Additionally, when the wearable article 200 is worn, the front waist portion 210 and the back waist portion 220 are connected to each other by fastening members such as tapes not shown in the drawings.

In the wearable article 200, the stretchable laminated sheet L is used for the front waist portion 210 and the back waist portion 220. The stretchable laminated sheet L is used for the front waist portion 210 and the back waist portion 220 in a state in which the aforementioned conveyance direction A and the direction around the waist coincide.

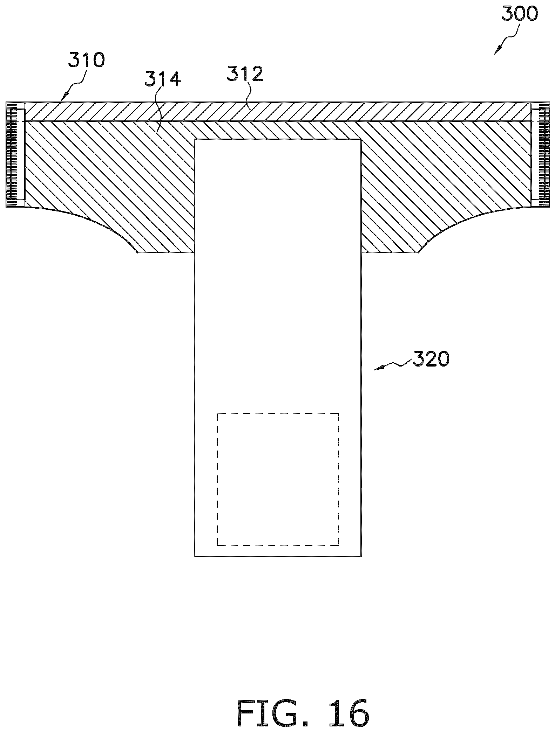

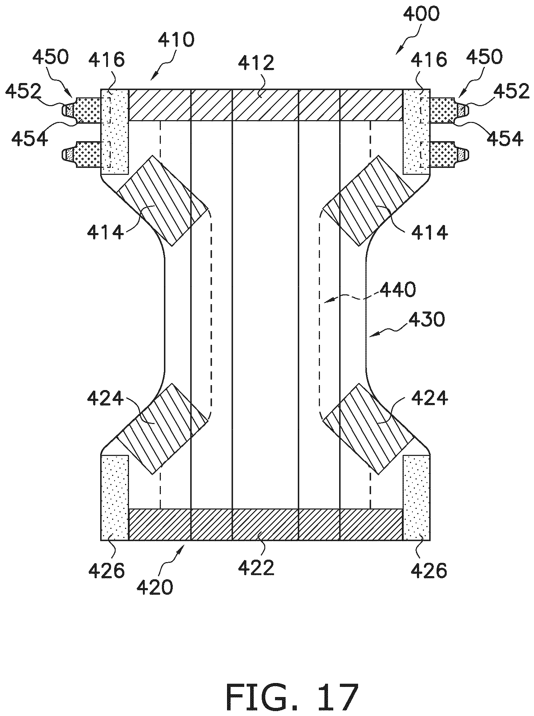

Specific examples of places where the stretchable laminated sheet L is used in wearable articles will now be described with reference to FIG. 16 and FIG. 17.

A wearable article 300 exemplified in FIG. 16 is a disposable diaper that is substantially T-shaped when it is flatly laid out (before it is worn). The wearable article 300 includes a single waist member 310 and an absorbent body 320 that is attached to the waist member 310.

The waist member 310 is a member disposed around the waist of the wearer. The waist member 310 includes a waistline portion 312 (the hatched portion with the positively sloping lines in FIG. 16) and a lower waist portion 314 (the hatched portion with the negatively sloping lines in FIG. 16). The waistline portion 312 is a portion disposed on the upper end of the waist member 310 and disposed around the waistline (covering the waistline) of the wearer (at a height position near the hip bones) when the wearable article 300 is worn. The lower waist portion 314 is a portion disposed below the waistline portion 312 and covers the region of the wearer's waist below the waist when the wearable article 300 is worn. The absorbent body 320 is attached to, in the left and right direction (width direction), the central portion of the lower waist portion 314 orthogonally to the lower waist portion 314.

It will be noted that the waistline portion 312 and the lower waist portion 314 may be separate members and joined together to form the waist member 310. Furthermore, the waistline portion 312 and the lower waist portion 314 may be formed integrally (by the same material).

The absorbent body 320 is a member joined to the waist member 310 and disposed in the crotch region of the wearer when the wearable article 300 is worn. The absorbent body 320 has an absorbent article not shown in the drawings, and the absorbent article mainly absorbs body fluids such as urine and sweat.

The stretchable laminated sheet L manufactured by the manufacturing method of the present disclosure is used for the waistline portion 312 and/or the lower waist portion 314 of the waist member 310 of the wearable article 300. This can realize the wearable article 300 having an improved breathability even in the portion(s) of the stretchable laminated sheet L.

It will be noted that in the stretchable laminated sheet L manufactured by the manufacturing method of the present disclosure, openings Op1 are also formed around the joints J between the pair of nonwoven fabric sheets S1, S2 and the film F not only in the joints J. For this reason, the stretchable laminated sheet L provided in the wearable article 300 has high breathability. Furthermore, because sufficient openings are provided in the stretchable laminated sheet L, the stretchable laminated sheet L that has high stretchability is obtained, and by using this, there is also obtained the advantageous effect that the wearable article 300 that easily fits the wearer is realized.

The wearable article 400 exemplified in FIG 17, like the wearable article 200 shown in FIG. 4, has a front waist portion 410, a back waist portion 420, a crotch portion 430, and an absorbent article 440. The crotch portion 430 and the absorbent article 440 are an example of the absorbent body in the claims. Moreover, the wearable article 400 has fastening tapes 450 serving as an example of connecting members.

The front waist portion 410 and the back waist portion 420 are both examples of a waist member disposed around the waist of the wearer. In the wearable article 400, the front waist portion 410 and the back waist portion 420 are spaced apart from each other. When the wearable article 400 is worn, the front waist portion 410 and the back waist portion 420 face each other.

The front waist portion 410 and the back waist portion 420 include waistline portions 412, 422 (the hatched portions with right-up straight lines in FIG. 17) and lower waist portions 414, 424 (the hatched portions with right-down straight lines in FIG. 17). The waistline portion 412 is a portion disposed on the upper end of the front waist portion 410 and disposed around the waistline of the front waist of the wearer (at a height position near the hip bones) when the wearable article 400 is worn. The waistline portion 422 is a portion disposed on the upper end of the back waist portion 420 and disposed around the waistline of the back waist of the wearer (near the hip bones) when the wearable article is worn. The lower waist portions 414 are portions disposed below the waistline portion 412 and cover the region of the wearer's front waist below the waist when the wearable article is worn. The lower waist portions 424 are portions disposed below the waistline portion 422 and cover the region of the wearer's back waist below the waist when the wearable article is worn.

The crotch portion 430 is attached to, in the left and right direction (width direction), central portion of the lower waist portions 414, 424 orthogonally to the lower waist portions 414, 424 and so as to straddle the lower waist portions 414, 424. That is, the crotch portion 430 is connected to the front waist portion 410 and the back waist portion 420. In other words, the front waist portion 410 and the back waist portion 420 are interconnected via the crotch portion 430.

The crotch portion 430 is a member disposed in the crotch region of the wearer when the wearable article is worn. The absorbent article 440 is disposed in the central portion of the crotch portion 430 so as to lie on the crotch portion 430. The absorbent article 440 is a member disposed in the crotch region of the wearer together with the crotch portion 430 when the wearable article 400 is worn.

It will be noted that the crotch portion 430 may be a member separate from the front waist portion 410 and the back waist portion 420 and joined to them to form the wearable article 400. Furthermore, the front waist portion 410, the back waist portion 420, and the crotch portion 430 may be formed integrally (by the same material).

In the width direction (left and right direction), both end portions of the front waist portion 410 are provided with attachment portions 416 (the hatched portions with dots in FIG. 17) of the fastening tapes 450 that are an example of connecting member attachment portions. It will be noted that the attachment portions 416 here mean not only the portions to which the fastening tapes 450 are attached but also the areas around the portions to which the fastening tapes 450 are attached.

The fastening tapes 450 include hook portions 452 provided with hook-shaped projections and coupling portions 454 (the hatched portions with crosses in FIG. 17) having one end side attached to the attachment portions 416 and the other end side provided with the hook portions 452. When the wearer wears the wearable article 400, the hook portions 452 of the fastening tapes 450 are connected (stuck) to loop portions (connected portions) 426 (the hatched portions with dots in FIG. 17) provided near both width direction (left and right direction) end portions of the back waist portion 420. It will be noted that the loop portions 426 are provided with numerous loop-shaped projections on which the hook-shaped projections of the hook portions 452 catch. It will be noted that the loop portions 426 may be members separate from the back waist portion 420 or may be formed by processing the back waist portion 420.

The stretchable laminated sheet L manufactured by the manufacturing method of the present disclosure is used for the waistline portion 412 and/or the lower waist portions 414 of the front waist portion 410, and/or the waistline portion 422 and/or the lower waist portions 424 of the back waist portion 420, in the wearable article 400. Furthermore, in addition to or instead of this, the stretchable laminated sheet L is used for the attachment portions (connecting member attachment portions) 416 of the front waist portion 410 and/or the loop portions (connected portions) 426 of the back waist portion 420. Furthermore, in addition to or instead of this, the stretchable laminated sheet L is used for the coupling portions 454 of the fastening tapes 450.

By using the stretchable laminated sheet L in this way, there can be realized the wearable article 400 that has improved breathability also in the stretchable laminated sheet L portion(s). It will be noted that in the stretchable laminated sheet L manufactured by the manufacturing method of the present disclosure, openings Op1 are formed not only around the joints J between the pair of nonwoven fabric sheets S1, S2 and the film F but also in the joints J. For this reason, the stretchable laminated sheet L provided in the wearable article 400 has high breathability. Furthermore, because sufficient openings are provided in the stretchable laminated sheet L, the stretchable laminated sheet L that has high stretchability is obtained, and by using this, there is also obtained the advantageous effect that the wearable article 400 that easily fits the wearer is realized.

### (1-2) Apparatus for Manufacturing Stretchable Laminated Sheet

Generally stated, the manufacturing apparatus 100 is the following kind of apparatus.

The manufacturing apparatus 100 manufactures the stretchable film F from a raw material. The manufacturing apparatus 100 conveys in an extended state the film F it has manufactured. Then, the manufacturing apparatus 100 causes the pair of nonwoven fabric sheets S1, S2 to merge with the conveyed film F and heat seals the pair of nonwoven fabric sheets S1, S2 and the film F in a state in which the film F in the extended state is disposed between the pair of nonwoven fabric sheets S1, S2 to thereby form the stretchable laminated sheet L. In other words, the manufacturing apparatus 100 causes the nonwoven fabric sheets S1, S2 to merge with the conveyed film F and heat seals the nonwoven fabric sheets S1, S2 and the film F in a state in which the film F in the extended state and the nonwoven fabric sheets S1, S2 are superimposed on top of each other to thereby form the stretchable laminated sheet L. Moreover, in order to improve the breathability of the stretchable laminated sheet L (specifically, the breathability of the film F of the stretchable laminated sheet L), the manufacturing apparatus 100 uses gear rolls 62, 64 to stretch (gear stretch) the stretchable laminated sheet L.

Details about the configuration of the manufacturing apparatus 100 are described below.

### (2) Details about Stretchable Laminated Sheet Manufacturing Apparatus

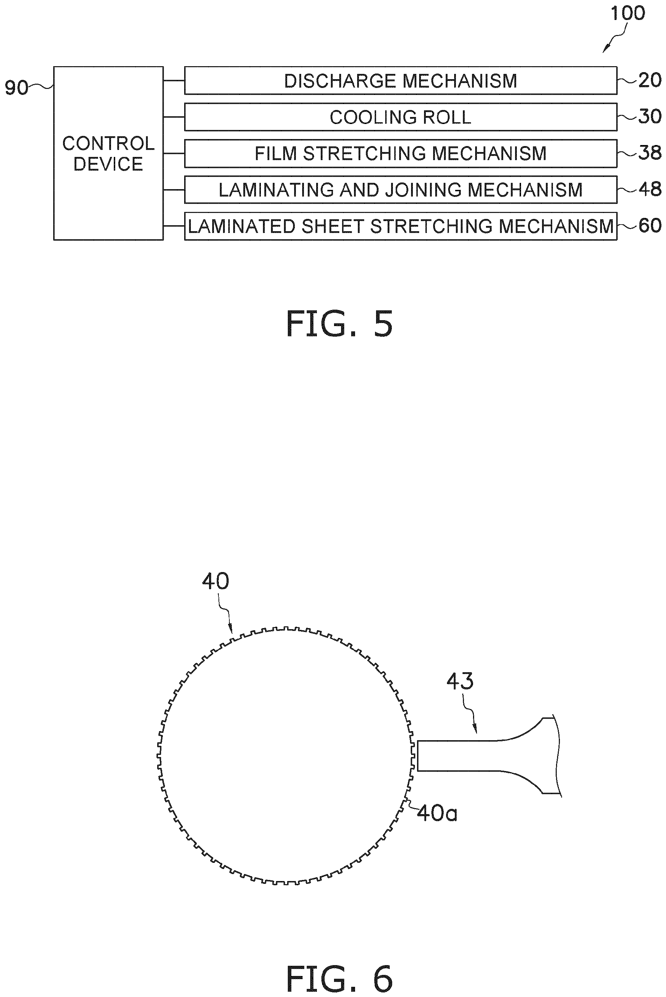

The manufacturing apparatus 100 of the stretchable laminated sheet L will now be described in greater detail with reference to FIG. 5 in addition to FIG. 1 to FIG. 4. FIG. 5 is a control block diagram of the manufacturing apparatus 100 of the stretchable laminated sheet L pertaining to the first embodiment.

The manufacturing apparatus 100, as shown in FIG. 1 and FIG. 5, mainly has a discharge mechanism 20, a cooling roll 30, a film stretching mechanism 38, a laminating and joining mechanism 48, a laminated sheet stretching mechanism 60, and a control device 90.

### (2-1) Discharge Mechanism

The discharge mechanism 20 is a device that forms a film-like intermediate Fa from a raw material (a resin material). The intermediate Fa is eventually formed into the film F. The discharge mechanism 20 specifically heats and melts the resin material to a temperature higher than the temperature range in which the resin material undergoes elastic deformation and discharges in a film-like shape the molten resin material from a discharge port 24 to form the film-like intermediate Fa. The film-like intermediate Fa formed by the discharge mechanism 20 is conveyed to the cooling roll 30, which is disposed below the discharge mechanism 20 as in FIG. 1.

It will be noted that the resin material is, for example, a material mainly comprising a thermoplastic elastic resin, and is a material that exhibits elasticity at room temperature. For example, though this is not intended to be limiting, as described in Patent Document 1 (International Publication No. 2019/150801), any of the thermoplastic elastomers prescribed in JIS K 6418:2007 (ISO 18064:2003) is used as the resin material.

### (2-2) Cooling Roll

The cooling roll 30 is a device that cools and solidifies the intermediate Fa to form the elastic (stretchable) film F.

The cooling roll 30 is driven to rotate by a drive device such as a motor not shown in the drawings. Inside the cooling roll 30 is formed a flow path, not shown in the drawings, through which flows a fluid for cooling the intermediate Fa.

As mentioned above, the intermediate Fa discharged from the discharge mechanism 20 is supplied to the cooling roll 30. It will be noted that the intermediate Fa discharged from the discharge port 24 of the discharge mechanism 20 is stretched in a first zone 80 (see FIG. 1) before reaching the cooling roll 30. In order to realize this, the cooling roll 30 rotates at a circumferential velocity greater than the feed rate of the resin material when the resin material is discharged from the discharge port 24 of the discharge mechanism 20 and stretches the film-like intermediate Fa until the thickness of the intermediate Fa reaches a predetermined value.

Furthermore, the cooling roll 30 is driven to rotate by the drive device and conveys the intermediate Fa along the outer peripheral surface of the cooling roll 30. The cooling roll 30 cools and solidifies, in a second zone 82 in which the intermediate Fa is in contact with the outer peripheral surface of the cooling roll 30, the intermediate Fa to the temperature range in which the resin material configuring the intermediate Fa undergoes elastic deformation. Because of this, the intermediate Fa turns into the film F in the second zone 82 and is drawn off the cooling roll 30. The film F drawn off the cooling roll 30 is fed via a guide roll 32 to the film stretching mechanism 38. The guide roll 32 may, like the cooling roll 30, have the function of cooling the film F.

It will be noted that the film F conveyed from the cooling roll 30 to the film stretching mechanism 38 is conveyed in a state in which it is extended by α% relative to the film F at its natural length (the length of the film F in a state in which no force is applied thereto).

### (2-3) Film Stretching Mechanism

The film stretching mechanism 38 is a device that further extends the film F conveyed thereto from the cooling roll 30 in the extended state.

The film stretching mechanism 38 mainly includes a drawing-off roll 34, a pinch roll 36, and an extension roll 40. The drawing-off roll 34 is driven to rotate by a drive device such as a motor not shown in the drawings. The extension roll 40 is driven to rotate by a drive device such as a motor not shown in the drawings.

The drawing-off roll 34 and the pinch roll 36 are adjacently disposed. The drawing-off roll 34 and the pinch roll 36 pinch the film F so that the film F conveyed from the cooling roll 30 to the drawing-off roll 34 does not slide along the outer peripheral surface of the drawing-off roll 34. The drawing-off roll 34 rotates synchronously with the cooling roll 30.

The film F is extended by the film stretching mechanism 38 in a third zone 84 from between the drawing-off roll 34 and the pinch roll 36 to until the film F reaches the extension roll 40. In order to realize this, the extension roll 40 rotates at a circumferential velocity faster than the circumferential velocity of the drawing-off roll 34 to extend the film F by β% relative to the film F at its natural length (the length of the film F in a state in which no force is applied thereto). β% is a value greater than α%. The film F that has been extended by β% relative to the film F at its natural length is conveyed along the outer peripheral surface of the extension roll 40.

### (2-4) Laminating and Joining Mechanism

The laminating and joining mechanism 48 forms a laminate body B in which the nonwoven fabric sheets S1, S2 and the film F that is in the extended state are superimposed. Particularly in the present embodiment, the laminating and joining mechanism 48 forms the laminate body B in which the film F in the extended state is disposed between the pair of nonwoven fabric sheets S1, S2. Moreover, the laminating and joining mechanism 48 functions as the joining unit in the claims and heat seals the nonwoven fabric sheets S1, S2 and the film F in the laminate body B to form the stretchable laminated sheet L. Particularly in the present embodiment, the laminating and joining mechanism 48 functions as the joining unit in the claims and heat seals the pair of nonwoven fabric sheets S1, S2 and the film F disposed between the pair of nonwoven fabric sheets S1, S2 in the laminate body B to form the stretchable laminated sheet L.

The extension roll 40, which is a part of the configuration of the film stretching mechanism 38, also functions as a part of the laminating and joining mechanism 48. Furthermore, the laminating and joining mechanism 48 has an ultrasonic bonding device 42.

The continuous first nonwoven fabric sheet S1, which is paid out from an original sheet roll not shown in the drawings, is supplied via a guide roll 50 to the extension roll 40 of the laminating and joining mechanism 48. Furthermore, the continuous second nonwoven fabric sheet S2, which is paid out from an original sheet roll not shown in the drawings, is supplied via guide rolls 52, 54 to the extension roll 40. In the laminating and joining mechanism 48, the extended film F conveyed along the extension roll 40 is sandwiched between the first nonwoven fabric sheet S1 and the second nonwoven fabric sheet S2 that are conveyed to the extension roll 40, and the laminate body B, in which the film F in the extended state is disposed between the pair of nonwoven fabric sheets S1, S2, is formed.

It will be noted that in a case where the stretchable laminated sheet L has a two-layer structure, for example the continuous first nonwoven fabric sheet S1 paid out from the original sheet roll not shown in the drawings is supplied via the guide roll 50 to the extension roll 40 of the laminating and joining mechanism 48. Then, in the laminating and joining mechanism 48, the laminate body B, in which the extended film F conveyed along the extension roll 40 is superimposed with the first nonwoven fabric sheet S1 conveyed to the extension roll 40, is formed.

The laminate body B moves in accompaniment with the rotation of the extension roll 40 and passes between the extension roll 40, which functions as an anvil roll, and a horn 43 of the ultrasonic bonding device 42. The surface of the extension roll 40 is provided with a plurality of protruding portions 40a that are arranged along the axial direction of rotation of the extension roll 40 and arranged along the circumferential direction of the extension roll 40 (see FIG. 6). Though this is not intended to limit their shape, the protruding portions 40a have rectangular shapes or oblong elliptical shapes extending such that their lengthwise direction coincides with the axial direction of rotation of the extension roll 40 when the protruding portions 40a are viewed in the radial direction toward the axis of rotation of the extension roll 40. However, the shapes of the protruding portions 40a described here are merely examples and may be other shapes.

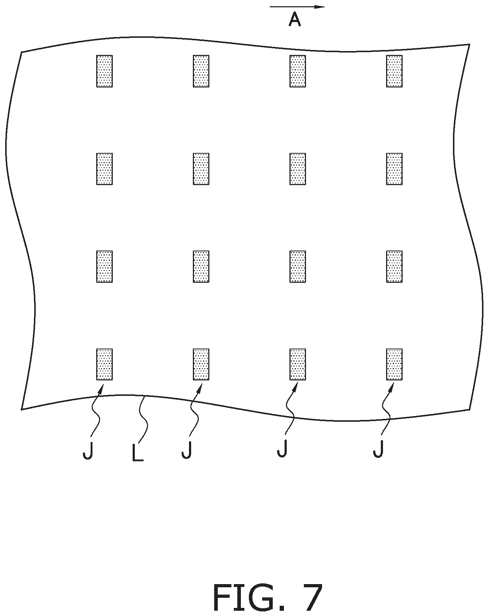

The extension roll 40 and the horn 43 are examples of the first joining member and the second joining member in the claims. The horn 43 is caused to vibrate by an ultrasonic oscillator not shown in the drawings in a state in which the laminate body B is sandwiched between the horn 43 and the extension roll 40 (more specifically, between the horn 43 and the protruding portions 40a of the extension roll 40) and ultrasonically seals the nonwoven fabric sheets S1, S2 (in particular, here, the pair of nonwoven fabric sheets S1, S2) and the film F. Ultrasonic sealing is one form of heat sealing. The stretchable laminated sheet L, in which the pair of nonwoven fabric sheets S1, S2 and the film F are intermittently joined together at the joints J, is formed as a result of the pair of nonwoven fabric sheets S1, S2 and the film F being ultrasonically sealed together, and is drawn off from the laminating and joining mechanism 48. In the stretchable laminated sheet L, as shown in FIG. 7 for example, the joints J (the dotted portions) are locally disposed. The stretchable laminated sheet L drawn off from the laminating and joining mechanism 48 is conveyed to the laminated sheet stretching mechanism 60 in the conveyance direction A that lies along the extension direction of the film F.

As described above, here, the laminating and joining mechanism 48 serving as an example of the joining unit ultrasonically seals the nonwoven fabric sheets S1, S2 and the film F. However, the mode of the heat sealing is not limited to ultrasonic sealing. For example, the laminating and joining mechanism 48 may sandwich the laminate body B between two rollers with built-in heaters and heat seal the nonwoven fabric sheets S1, S2 and the film F in a way as in FIG. 7 for example.

Furthermore, here, as mentioned above, the extension roll 40, which is a part of the configuration of the film stretching mechanism 38, also functions as an anvil roll in the laminating and joining mechanism 48. However, the laminating and joining mechanism 48 is not limited to this and may have an anvil roll separate from the extension roll 40 that forms the laminate body B.

### (2-5) Laminated Sheet Stretching Mechanism

The laminated sheet stretching mechanism 60 is an example of the stretcher in the claims. The laminated sheet stretching mechanism 60 uses gear rolls 62, 62 to stretch (gear stretch) the stretchable laminated sheet L.

Specifically, the laminated sheet stretching mechanism 60 has a pair of gear rolls 62, 64 having recesses and projections in their peripheral surfaces. More specifically, in the gear roll 62, pluralities of tooth portions 62a and valley portions 62b are alternately provided along the circumferential direction of the gear roll 62 as in a spur gear. In the gear roll 64 also, pluralities of tooth portions 64a and valley portions 64b are alternately provided along the circumferential direction of the gear roll 64 as in a spur gear. Additionally, the gear roll 62 and the gear roll 64 are rotated by a drive mechanism such as a motor not shown in the drawings, whereby the tooth portions 62a of the gear roll 62 and the valley portions 64b of the gear roll 64, and the valley portions 62b of the gear roll 62 and the tooth portions 64a of the gear roll 64, mesh with each other.

The stretchable laminated sheet L conveyed along the conveyance direction A from the laminating and joining mechanism 48 is supplied to the place where the gear roll 62 and the gear roll 64 mesh and is stretched. As a result of the stretchable laminated sheet L being stretched by the laminated sheet stretching mechanism 60, the film F becomes extended by γ% relative to the film F at its natural length (the length of the film F in a state in which no force is applied thereto). The stretchable laminated sheet L that has been extended by the laminated sheet stretching mechanism 60 (the stretchable laminated sheet L in which the film F configuring the stretchable laminated sheet L has been extended by γ% relative to the film F at its natural length) is conveyed by a downstream conveyance mechanism 70 while maintaining its extension ratio and is conveyed to a subsequent process (a wearable article manufacturing process neither shown in the drawings nor described).

It will be noted that γ% is a value greater than β%, though this is not intended to be limiting. That is, the film F (also including the film F included in the stretchable laminated sheet L) may be extended by extension ratios of α%, β (>α) %, γ (>β) % relative to the film F at its natural length by the second zone 82, the third zone 84, and the laminated sheet stretching mechanism 60, respectively. In other words, although here the film F is extended a relatively great extent relative to the film F at its natural length by the laminated sheet stretching mechanism 60, damage to the film F is easily reduced even when the film F is extended a relatively great extent because the film F is reinforced as a result of being joined to the nonwoven fabric sheets S1, S2.

Because the stretchable laminated sheet L is stretched by the laminated sheet stretching mechanism 60, openings with a sufficient area can be formed in and/or around the places where the nonwoven fabric sheets S1, S2 and the film F have been heat sealed, and the stretchable laminated sheet L that has improved breathability can be manufactured.

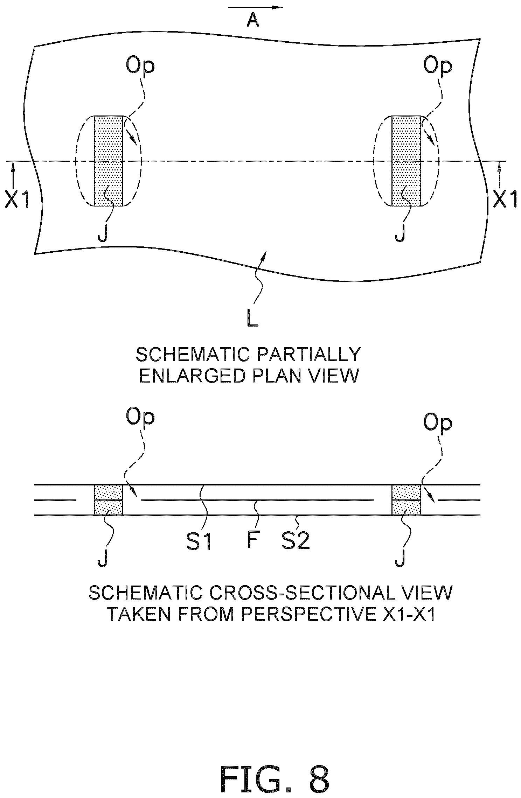

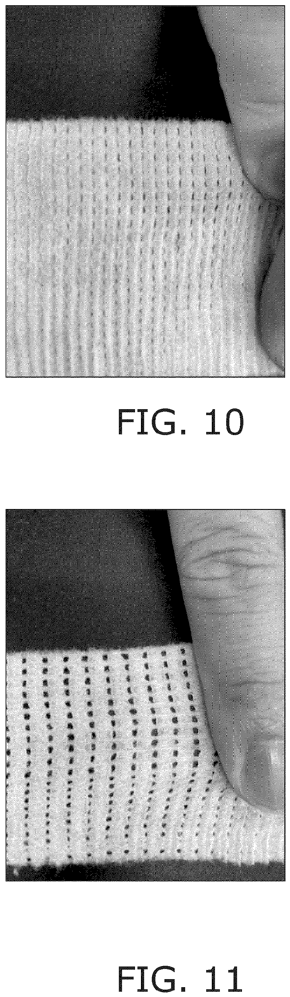

Specifically, when the stretchable laminated sheet L is extended in the conveyance direction A before stretching is performed by the laminated sheet stretching mechanism 60, as the film F is severed around the joints J (the dotted portions) of the stretchable laminated sheet L, openings Op are formed around the joints J so as shown in FIG. 8. However, these openings Op are relatively small as can be seen from the photograph in FIG. 10, and openings Op with a sufficient area for ensuring the breathability of the stretchable laminated sheet L may not be ensured.

In contrast, by stretching with the laminated sheet stretching mechanism 60, the film F becomes severed even for example in some of the joints J of the stretchable laminated sheet L, and as shown in FIG. 8 the film F becomes severed and openings Op1 are also formed in the joints J (the dotted portions) of the stretchable laminated sheet L in addition to the openings Op around the joints J. As a result, when the stretchable laminated sheet L is extended in the conveyance direction A, relatively large openings become formed in the stretchable laminated sheet L as shown in FIG. 8. It will be noted that the openings formed as a result of the laminated sheet stretching mechanism 60 performing stretching are not limited to being formed in the joints J, and there are also cases where they are formed around the joints J (formed such that the openings Op become larger). In this way, by performing gear stretching on the stretchable laminated sheet L, improved breathability in the stretchable laminated sheet L (the film F of the stretchable laminated sheet L) can be realized.

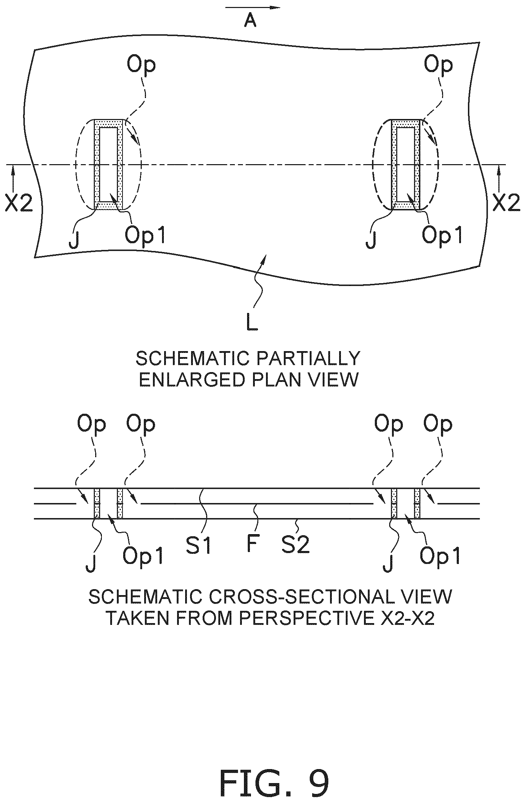

It will be noted that FIGS. 8 and 9 are merely drawings for explanation and that the shapes and dimensions of the openings Op, Op1 shown in FIGS. 8 and 9 are not intended to limit the shapes of the openings that are actually formed.

It will be noted that when the stretchable laminated sheet L configured by the nonwoven fabrics and the thermoplastic elastic resin or the like is sandwiched by the metal gear rolls 62, 64 in the laminated sheet stretching mechanism 60, the stretchable laminated sheet L may become charged with static electricity. If the stretchable laminated sheet L becomes charged with static electricity, the static electricity may adversely affect the process in which the stretchable laminated sheet L is processed to manufacture the wearable articles. For that reason, it is preferred that the stretchable laminated sheet L be brought into contact with a grounded conductor on the downstream side of the laminated sheet stretching mechanism 60 in the conveyance direction A of the stretchable laminated sheet L.

### (2-6) Control Device

The control device 90 has a CPU, memories such as a ROM and a RAM, an input/output device, and various types of electrical components and electronic components not shown in the drawings.

The control device 90 is, as shown in FIG. 5, electrically connected to the discharge mechanism 20, the cooling roll 30, the film stretching mechanism 38, the laminating and joining mechanism 48, and the laminated sheet stretching mechanism 60. The control device 90, as a result of the CPU executing programs stored in the memories, controls the discharge mechanism 20, the cooling roll 30, the film stretching mechanism 38, the laminating and joining mechanism 48, and the laminated sheet stretching mechanism 60 to operate in concert with each other.

It will be noted that the control device 90 may be realized by software, realized by hardware (various types of electrical circuits and electronic circuits), or realized by cooperation between software and hardware as long as it can exhibit the functions described here.

### (3) Method of Manufacturing Stretchable Laminated Sheet

A method of manufacturing the stretchable laminated sheet L will now be described.

First, the formation of the film F will be described.

In the discharge mechanism 20, for example, the resin material exhibiting elasticity at room temperature is heated and melted and is discharged as the film-like intermediate Fa from the discharge port 24. Thereafter, the intermediate Fa is cooled and solidified by the cooling roll 30, and the elastic (stretchable) film F is formed. In other words, here, the discharge mechanism 20 and the cooling roll 30 function as the former in the claims, and the film F is formed from the resin material serving as a raw material. It will be noted that on the cooling roll 30 the film F is stretched to a state in which it is extended by α% relative to the film F at its natural length (the length of the film F in a state in which no force is applied thereto).

The film F drawn off from the cooling roll 30 and conveyed in the extended state from the cooling roll 30 is further extended by the film stretching mechanism 38. Specifically, in the film stretching mechanism 38, the film F is stretched to a state in which it is extended by β (>α) % relative to the film F at its natural length.

Then, in the laminating and joining mechanism 48, the laminate body B, in which the nonwoven fabric sheets S1, S2 paid out from the original sheet rolls not shown in the drawings and the film F that is in the extended state are superimposed, is formed. Particularly in the present embodiment, in the laminating and joining mechanism 48, the laminate body B, in which the film F in the extended state is disposed between the first nonwoven fabric sheet S1 paid out from the original sheet roll not shown in the drawings and the second nonwoven fabric sheet S2 paid out from the original sheet roll not shown in the drawings, is formed. Moreover, the laminating and joining mechanism 48 sandwiches the laminate body B between the extension roll 40 and the horn 43 of the ultrasonic bonding device 42 and heat seals the pair of nonwoven fabric sheets S1, S2 and the film F to form them into the stretchable laminated sheet L.

Next, the laminated sheet stretching mechanism 60 stretches (gear stretches) the stretchable laminated sheet L, which is conveyed thereto along the conveyance direction A from the laminating and joining mechanism 48 and is conveyed between the pair of gear rolls 62, 64. Specifically, the laminated sheet stretching mechanism 60 stretches the film F to a state in which it is extended by γ% relative to the film F at its natural length. For example, γ% is a value greater than β%. As a result of the gear stretching, openings with a sufficient area become formed in and/or around the places in the stretchable laminated sheet L where the nonwoven fabric sheets S1, S2 and the film F have been heat sealed.

### (4) Characteristics

(4-1)
The manufacturing apparatus 100 of the stretchable laminated sheet L is the apparatus 100 for manufacturing the stretchable laminated sheet L in which the nonwoven fabric sheets S1, S2 serving as examples of substrate sheets and the stretchable film F are disposed on top of each other and in which the nonwoven fabric sheets S1, S2 and the film F are joined together. The manufacturing apparatus 100 of the stretchable laminated sheet L includes the laminating and joining mechanism 48 serving as an example of a joining unit and the laminated sheet stretching mechanism 60 serving as an example of a stretcher. The laminating and joining mechanism 48 has an extension roll 40 (anvil roll) and a horn 43 serving as examples of a first joining member and a second joining member. The laminating and joining mechanism 48 sandwiches between the extension roll 40 and the horn 43 the laminate body B in which the nonwoven fabric sheets S1, S2 and the film F that is in an extended state are superimposed and heat seals the nonwoven fabric sheets S1, S2 and the film F to form the stretchable laminated sheet L. The laminated sheet stretching mechanism 60 has the pair of gear rolls 62, 64 having recesses and projections in their peripheral surfaces. The laminated sheet stretching mechanism 60 stretches the stretchable laminated sheet L which is conveyed between the pair of gear rolls 62, 64.

The manufacturing apparatus 100 of the stretchable laminated sheet L can form openings with a sufficient area in and/or around the places where the nonwoven fabric sheets S1, S2 and the film F have been heat sealed by heat sealing the nonwoven fabric sheets S1, S2 and the film F to form the stretchable laminated sheet L and then performing stretching with the gear rolls 62, 64 having recesses and projections in their peripheral surfaces. Therefore, it can manufacture the stretchable laminated sheet L that has improved breathability.

(4-2)
The manufacturing apparatus 100 of the stretchable laminated sheet L includes the discharge mechanism 20 and a cooling roll 30 that function as a former and the film stretching mechanism 38 that serves as an example of a film stretcher. The former forms the film F from a raw material (a resin material). The film stretching mechanism 38 is disposed between the former and the laminating and joining mechanism 48 in the conveyance direction A of the film F and stretches the film F. The extension ratio of the film F conveyed between the film stretching mechanism 38 and the laminating and joining mechanism 48 to the film F at its natural length is greater than the extension ratio of the film F conveyed between the former and the film stretching mechanism 38 to the film F at its natural length. Furthermore, the extension ratio of the film F of the stretchable laminated sheet L in the laminated sheet stretching mechanism 60 to the film F at its natural length is greater than the extension ratio of the film F conveyed between the film stretching mechanism 38 and the laminating and joining mechanism 48 to the film F at its natural length.

In this manufacturing apparatus 100 of the stretchable laminated sheet L, the thickness of the film F can be reduced by extending the film F in stages. In particular, in this manufacturing apparatus 100 of the stretchable laminated sheet L, by using the film F to form the stretchable laminated sheet L and thereafter further stretching the film F, the thickness of the film F can be reduced while reducing damage to the film F.

(4-3)
The stretchable laminated sheet L manufacturing method is a method of manufacturing the stretchable laminated sheet L in which the nonwoven fabric sheets S1, S2 serving as examples of substrate sheets and the stretchable film F are disposed on top of each other and in which the nonwoven fabric sheets S1, S2 and the film F are joined together. The stretchable laminated sheet L manufacturing method includes a sealing step and a stretching step. In the sealing step, the nonwoven fabric sheets S1, S2 and the film F that is in an extended state are heat sealed to form the stretchable laminated sheet L. In the stretching step, the stretchable laminated sheet L which is conveyed between the pair of gear rolls 62, 64 having recesses and projections in their peripheral surfaces is stretched by the pair of gear rolls 62, 64.

This stretchable laminated sheet L manufacturing method can form openings with a sufficient area in and/or around the places where the nonwoven fabric sheets S1, S2 and the film F have been heat sealed by heat sealing the nonwoven fabric sheets S1, S2 and the film F to form the stretchable laminated sheet L and then performing stretching with the gear rolls 62, 64 having recesses and projections in their peripheral surfaces. Therefore, it can manufacture the stretchable laminated sheet L that has improved breathability.

### < Second Embodiment >

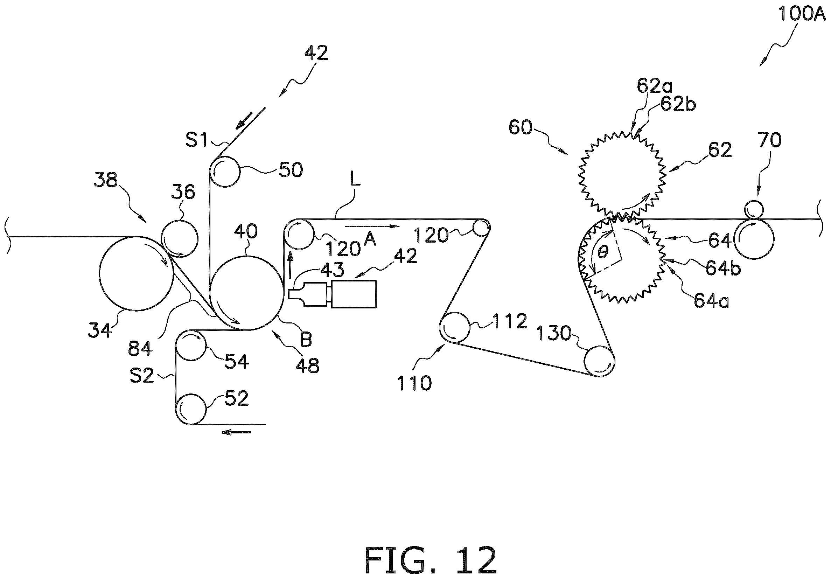

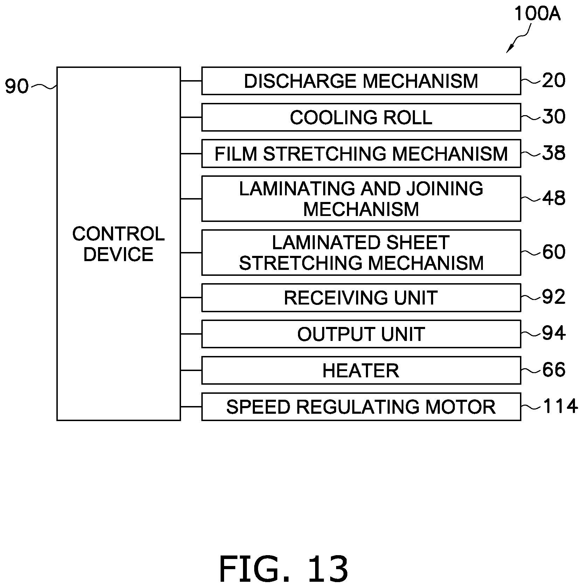

A manufacturing apparatus 100A of the stretchable laminated sheet L pertaining to a second embodiment will be described below with reference to FIG. 12 and FIG. 13. FIG. 12 is a schematic diagram showing the configuration of the manufacturing apparatus 100A of the stretchable laminated sheet L, with illustration of the former (the discharge mechanism 20, the cooling roll 30, etc.) of the film being omitted. FIG. 13 is an example of a control block diagram of the manufacturing apparatus 100A of the stretchable laminated sheet L.

It will be noted that the manufacturing apparatus 100A of the second embodiment differs from the manufacturing apparatus 100 of the first embodiment mainly in the following configuration "a" to configuration "c". It will be noted that the manufacturing apparatus 100A does not need to have all of configuration "a" to configuration "c". The manufacturing apparatus 100A may have any one of configuration "a" to configuration "c" or two of configuration "a" to configuration "c".
· Configuration "a": An adjuster 110 that adjusts the conveyance speed of the stretchable laminated sheet L supplied to the laminated sheet stretching mechanism 60 is provided between the laminating and joining mechanism 48 and the laminated sheet stretching mechanism 60 on the conveyance path of the stretchable laminated sheet L, and the control device 90 functions as a speed controller that controls the operation of the adjuster 110.
· Configuration b: The manufacturing apparatus 100A includes a heater 66 that heats at least one of the pair of gear rolls 62, 64 (in the present embodiment, at least the gear roll 64 includes the heater 66).
· Configuration c: The manufacturing apparatus 100A has a receiving unit 92 that receives information relating to the size of the opening that the laminated sheet stretching mechanism 60 is to form in the stretchable laminated sheet L, and the control device 90 determines settings of the gear rolls 62, 64 in the laminated sheet stretching mechanism 60 based on the information.

Here, just configuration a to configuration "c", which are the differences the manufacturing apparatus 100A of the second embodiment has from the manufacturing apparatus 100 of the first embodiment, will be described, and description relating to similarities will be omitted unless necessary. Furthermore, since the stretchable laminated sheet L that the manufacturing apparatus 100A manufactures is the same as the stretchable laminated sheet L that the manufacturing apparatus 100 manufactures, description of the stretchable laminated sheet L will also be omitted here.

### (1) Details about Configuration "a" to Configuration "c"

### (1-1) Configuration "a"

Configuration "a" will now be described.

First, the reason for providing configuration "a" will be described.

As described in the first embodiment, the stretchable laminated sheet L that has been formed by the laminating and joining mechanism 48 is gear stretched in the laminated sheet stretching mechanism 60, and openings are formed therein.

The size of the opening formed in the laminated sheet stretching mechanism 60 varies depending on how much the stretchable laminated sheet L is stretched by the laminated sheet stretching mechanism 60. Additionally, how much the stretchable laminated sheet L is stretched by the laminated sheet stretching mechanism 60 also varies depending on how much tension is acting on the stretchable laminated sheet L supplied to the laminated sheet stretching mechanism 60. Specifically, even if the operating conditions of the laminated sheet stretching mechanism 60 do not particularly change, in a case where a relatively large tension has acted on the stretchable laminated sheet L supplied to the laminated sheet stretching mechanism 60, relatively large openings tend to be formed in the stretchable laminated sheet L, and in a case where only a relatively small tension is acting on the stretchable laminated sheet L supplied to the laminated sheet stretching mechanism 60, only relatively small openings are formed in the stretchable laminated sheet L.

Furthermore, in a case where excessive tension is acting on the stretchable laminated sheet L supplied to the laminated sheet stretching mechanism 60, there is a possibility for the stretchable laminated sheet L to sustain damage when it is stretched by the laminated sheet stretching mechanism 60.

For that reason, in order to form openings with generally the same size in the entire stretchable laminated sheet L and suppress damage to the stretchable laminated sheet L, it is preferred that the tension acting on the stretchable laminated sheet L supplied to the laminated sheet stretching mechanism 60 be controlled to an appropriate value.

Additionally, since the tension acting on the stretchable laminated sheet L varies depending on the conveyance speed of the stretchable laminated sheet L supplied to the laminated sheet stretching mechanism 60, the manufacturing apparatus 100A of the second embodiment includes the adjuster 110 that is provided between the laminating and joining mechanism 48 and the laminated sheet stretching mechanism 60 and adjusts the conveyance speed of the stretchable laminated sheet L supplied to the laminated sheet stretching mechanism 60.

The adjuster 110 mainly has a speed adjusting roller 112 serving as an example of a second roller around which the stretchable laminated sheet L is wound and a speed regulating motor 114 that drives the speed adjusting roller 112 to rotate (see FIG. 12 and FIG. 13). The speed regulating motor 114 is electrically connected by the control device 90, and the operation of the speed regulating motor 114 (operating/stopped, rotational speed, etc.) is controlled by the control device 90. The speed adjusting roller 112 is a roller whose outer surface, around which the stretchable laminated sheet L is wound, has a high adhesion or coefficient of friction. Specifically, the adhesion or coefficient of friction of the outer surface of the speed adjusting roller 112 is higher than the adhesion or coefficient of friction of the outer surfaces of guide rollers 120 serving as an example of first roller around which the stretchable laminated sheet L is wound. It will be noted that the guide rollers 120 are rollers that are disposed between the laminating and joining mechanism 48 and the adjuster 110 on the conveyance path of the stretchable laminated sheet L and guide the stretchable laminated sheet L wound around them. The rollers are, for example, freely rotating rollers.

The adhesion of the outer surface of the speed adjusting roller 112 is realized by, for example, sticking an adhesive material such as rubber on the outer surface of the speed adjusting roller 112. Furthermore, the relatively high coefficient of friction of the outer surface of the speed adjusting roller 112 is realized by, for example, sticking a member with a high coefficient of friction on the outer surface of the speed adjusting roller 112 or performing a process to roughen the outer surface of the speed adjusting roller 112.

The adjuster 110 varies the conveyance speed of the stretchable laminated sheet L in the adjuster 110 and, as a result, varies the conveyance speed of the stretchable laminated sheet L supplied to the laminated sheet stretching mechanism 60, by varying the rotational speed of the speed adjusting roller 112 having the outer surface with a relatively high adhesion or coefficient of friction.

It will be noted that, rather than having a configuration using the single speed adjusting roller 112, the adjuster 110 may use two rollers to sandwich the stretchable laminated sheet L and adjust the conveyance speed of the stretchable laminated sheet L.

The adjustment of the conveyance speed of the stretchable laminated sheet L supplied to the laminated sheet stretching mechanism 60 by the adjuster 110, and the adjustment of the tension acting on the stretchable laminated sheet L supplied to the laminated sheet stretching mechanism 60 that is obtained as a result, will now be described.

For example, assuming that the conveyance mechanism 70 is conveying the stretchable laminated sheet L at a conveyance speed V and that the adjuster 110 is also conveying the stretchable laminated sheet L at the conveyance speed V.

In this condition, further assuming that the conveyance speed V at which the conveyance mechanism 70 conveys the stretchable laminated sheet L is not changed but the rotational speed of the speed regulating motor 114 is decreased so that the rotational speed of the speed adjusting roller 112 is decreased. In this case, since the adjuster 110 conveys the stretchable laminated sheet L at a relatively low speed and the conveyance mechanism 70 conveys the stretchable laminated sheet L at a relatively high speed, the tension acting on the stretchable laminated sheet L becomes greater between the adjuster 110 and the conveyance mechanism 70. As a result, the tension acting on the stretchable laminated sheet L supplied to the laminated sheet stretching mechanism 60 becomes smaller relative to the tension acting on the stretchable laminated sheet L conveyed from the laminating and joining mechanism 48 to the adjuster 110.

Conversely, assuming that, under the condition where the conveyance mechanism 70 is conveying the stretchable laminated sheet L at the conveyance speed V and the adjuster 110 is also conveying the stretchable laminated sheet L at the conveyance speed V, the conveyance speed V at which the conveyance mechanism 70 conveys the stretchable laminated sheet L is not changed but the rotational speed of the speed regulating motor 114 is increased so that the rotational speed of the speed adjusting roller 112 is increased. In this case, since the adjuster 110 conveys the stretchable laminated sheet L at a relatively high speed and the conveyance mechanism 70 conveys the stretchable laminated sheet L at a relatively low speed, the tension acting on the stretchable laminated sheet L becomes smaller between the adjuster 110 and the conveyance mechanism 70. As a result, the tension acting on the stretchable laminated sheet L supplied to the laminated sheet stretching mechanism 60 becomes greater relative to the tension acting on the stretchable laminated sheet L conveyed from the laminating and joining mechanism 48 to the adjuster 110.

A mode of controlling the tension acting on the stretchable laminated sheet L supplied to the laminated sheet stretching mechanism 60 during the operation of the manufacturing apparatus 100A will now be described.

First, when the manufacturing apparatus 100A starts operating or when the manufacturing apparatus 100A stops operating, the conveyance speed of the film F and the stretchable laminated sheet L is slow compared with that of during steady operation. It will be noted that steady operation means a state in which a predetermined period has elapsed since the manufacturing apparatus 100A has started operating and the conveyance speed of the film F and the stretchable laminated sheet L has reached a constant speed. For this reason, when the manufacturing apparatus 100A starts operating or when the manufacturing apparatus 100A stops operating, the tension acting on the stretchable laminated sheet L supplied to the laminated sheet stretching mechanism 60 may become smaller such that only relatively small openings are formed in the stretchable laminated sheet L.

Thus, it is preferred that, when the manufacturing apparatus 100A starts operating or when the manufacturing apparatus 100A stops operating, the control device 90 serving as a speed controller control the operation of the adjuster 110 (more specifically, the operation of the speed regulating motor 114) to reduce the conveyance speed of the stretchable laminated sheet L in the adjuster 110 and thereby make the tension acting on the stretchable laminated sheet L supplied to the laminated sheet stretching mechanism 60 greater relative to the tension acting on the stretchable laminated sheet L conveyed from the laminating and joining mechanism 48 to the adjuster 110.

Meanwhile, during steady operation of the manufacturing apparatus 100A, since the conveyance speed of the film F and the stretchable laminated sheet L relatively increases, the tension acting on the stretchable laminated sheet L supplied to the laminated sheet stretching mechanism 60 may become too large such that the openings formed in the stretchable laminated sheet L become too large or the stretchable laminated sheet L sustains damage.

Thus, it is preferred that, when the manufacturing apparatus 100A starts operating or when the manufacturing apparatus 100 stops operating, the control device 90 serving as a speed controller control the operation of the adjuster 110 (more specifically, the operation of the speed regulating motor 114) to increase the conveyance speed of the stretchable laminated sheet L in the adjuster 110 and thereby make the tension acting on the stretchable laminated sheet L supplied to the laminated sheet stretching mechanism 60 smaller relative to the tension acting on the stretchable laminated sheet L conveyed from the laminating and joining mechanism 48 to the adjuster 110.

It will be noted that, preferably, it is preferred that the control device 90 serving as a speed controller control the operation of the adjuster 110 (more specifically, the operation of the speed regulating motor 114) to always keep the tension acting on the stretchable laminated sheet L supplied to the laminated sheet stretching mechanism 60 constant.

### (1-2) Configuration "b"

Configuration "b" will now be described.

If the stretchable laminated sheet L is at a low temperature when it is gear stretched in the laminated sheet stretching mechanism 60, the thermoplastic film F of the stretchable laminated sheet L for example may harden and become difficult to stretch.

Thus, at least one of the pair of gear rolls 62, 64 (in the present embodiment, at least the gear roll 64) is provided with a heater 66 that heats the gear rolls 62, 64 to a predetermined temperature. By using the heater 66 to heat the gear rolls 62, 64, the stretchable laminated sheet L that contacts them is also heated and becomes easier to stretch.

It will be noted that it is preferred that the stretchable laminated sheet L be guided between the pair of gear rolls 62, 64 after contacting for a predetermined length the outer peripheral surface of the one (here, the gear roll 64) of the pair of gear rolls 62, 64 that is heated by the heater 66, so that the stretchable laminated sheet L is easily heated. The stretchable laminated sheet L is, for example, guided between the pair of gear rolls 62, 64 after contacting the outer peripheral surface of the gear roll 64 for 5% or more of the outer peripheral length of the gear roll 64, though this is not intended to limit the numerical value.

In order to allow the stretchable laminated sheet L to contact the outer peripheral surface of the gear roll 64 before the stretchable laminated sheet L is guided between the pair of gear rolls 62, 64, the stretchable laminated sheet L conveyed from the adjuster 110 (in a case where the adjuster 110 is not provided, the stretchable laminated sheet L conveyed from the laminated sheet stretching mechanism 60) is not directly guided between the gear rolls 62, 64. Specifically, as in FIG. 12, the stretchable laminated sheet L conveyed from the adjuster 110 (in a case where the adjuster 110 is not provided, the stretchable laminated sheet L conveyed from the laminated sheet stretching mechanism 60) is guided by a guide roller 130 so that it is wound around the outer peripheral surface of the gear roll 64, and thereafter it is guided between the gear rolls 62, 64.

### (1-3) Configuration "c"

Configuration "c" will now be described.

First, the reason for providing configuration "c" will be described.

The size of the opening required in the stretchable laminated sheet L are not always constant and may vary depending on, for example, the wearable articles 200, 300, 400 in which the stretchable laminated sheet L is used. In order to change the size of the opening that the laminated sheet stretching mechanism 60 forms in the stretchable laminated sheet L in accordance with the size of the opening required in the stretchable laminated sheet L, it is preferred to change the settings of the gear rolls 62, 64 of the laminated sheet stretching mechanism 60 for example. It will be noted that the settings of the gear rolls 62, 64 are, for example, the tooth depth of the gear rolls 62, 64 (the distance from the tooth roots to the tooth tips; here, the distance between the valley portions 62b and the tops of the tooth portions 62a) and the distance between the axial center of the gear roll 62 and the axial center of the gear roll 64. The larger the tooth depth of the gear rolls 62, 64 is, the larger the size of the opening that the laminated sheet stretching mechanism 60 forms in the stretchable laminated sheet L becomes, and the smaller the distance between the axial center of the gear roll 62 and the axial center of the gear roll 64 is, the larger the size of the opening that the laminated sheet stretching mechanism 60 forms in the stretchable laminated sheet L becomes.

However, the settings of the gear rolls 62, 64 are not limited to those that have been exemplified and may also be a property of each of the gear rolls 62, 64, or properties relating to correlations between the gear roll 62 and the gear roll 64, that vary the amount of stretching by the stretchable laminated sheet L. The operator of the manufacturing apparatus 100A may change the settings of the gear rolls 62, 64 by trial and error, but in this case, it may take time to change the settings.

Thus, it is preferred that the control device 90 of the manufacturing apparatus 100A determines the settings of the gear rolls 62, 64 in the laminated sheet stretching mechanism 60 based on information relating to the size of the opening that the laminated sheet stretching mechanism 60 is to form in the stretchable laminated sheet L. For that reason, the control device 90 of the manufacturing apparatus 100A has configuration "c".

Configuration "c" will now be specifically described.

The manufacturing apparatus 100A has a receiving unit 92 that receives information relating to the size of the opening that the laminated sheet stretching mechanism 60 is to form in the stretchable laminated sheet L. The receiving unit 92 is, for example, a switch or a touch panel that receives input of information by the operator of the manufacturing apparatus 100A. Furthermore, the receiving unit 92 may be a communication device that receives information sent via a network such as the internet from a terminal (e.g., a computer serving as a management device that manages the manufacturing apparatus 100A or a portable terminal carried by the operator) operated by the operator of the manufacturing apparatus 100A.

It will be noted that the information relating to the size of the opening that the laminated sheet stretching mechanism 60 is to form in the stretchable laminated sheet L is, for example, a numerical value representing the ratio of the desired opening size to the sealing area in which the laminating and joining mechanism 48 seals the nonwoven fabric sheets S1, S2 and the film F. Furthermore, the information relating to the size of the opening that the laminated sheet stretching mechanism 60 is to form in the stretchable laminated sheet L may, for example, also be the numerical value itself of the desired opening size. Furthermore, the information relating to the size of the opening that the laminated sheet stretching mechanism 60 is to form in the stretchable laminated sheet L may also, for example, be relative indicators indicating the desired opening size, such as "large", "medium", and "small".

Additionally, the control device 90 of the manufacturing apparatus 100A functions as a determining unit in the claims and determines the settings of the gear rolls 62, 64 in the laminated sheet stretching mechanism 60 based on the information, which has been received by the receiving unit 92, relating to the size of the opening that the laminated sheet stretching mechanism 60 is to form in the stretchable laminated sheet L.

In order to realize this, for example, the memories of the control device 90 store correlation information in which the size of the opening that the laminated sheet stretching mechanism 60 is to form in the stretchable laminated sheet L and the settings of the gear rolls 62, 64 are correlated with each other. The correlation information is information based on test results obtained by using a test device for testing the manufacturing apparatus 100A, gear stretching the stretchable laminated sheet L while changing the settings of the gear rolls 62, 64, and measuring the openings formed by the gear stretching. Furthermore, the correlation information is information relating to correlations between the settings of the gear rolls 62, 64 and the openings formed by the gear stretching, which is calculated theoretically or obtained by computer simulation.

The control device 90 references the correlation information to determine the settings of the gear rolls 62, 64 corresponding to the information, received by the receiving unit 92, relating to the size of the opening that the laminated sheet stretching mechanism 60 is to form in the stretchable laminated sheet L.

The manufacturing apparatus 100Ahas, for example, a display serving as an output unit 94, and the control device 90 displays on the display serving as the output unit 94 the settings of the gear rolls 62, 64 on which it has determined. Alternatively, the manufacturing apparatus 100A may, for example, have a communication device serving as the output unit 94, and the control device 90 may send the settings of the gear rolls 62, 64 on which it has determined to a terminal (e.g., a computer serving as a management device that manages the manufacturing apparatus 100A or a portable terminal carried by the operator) operated by the operator of the manufacturing apparatus 100A.

By configuring the manufacturing apparatus 100A in this way, the operator of the manufacturing apparatus 100A can replace the gear rolls 62, 64 used in the laminated sheet stretching mechanism 60 or regulate the distance between the axial center of the gear roll 62 and the axial center of the gear roll 64 based on the settings of the gear rolls 62, 64 that the control device 90 outputs from the output unit 94.

### (2) Characteristics

The manufacturing apparatus 100A of the second embodiment has the following characteristics in addition to the characteristics described in "(4) Characteristics" of the manufacturing apparatus 100 of the first embodiment.

(2-1)
The manufacturing apparatus 100A of the stretchable laminated sheet L includes the adjuster 110 and the control device 90 serving as a speed controller. The adjuster 110 is disposed between the laminating and joining mechanism 48 and the laminated sheet stretching mechanism 60 on the conveyance path of the stretchable laminated sheet L and adjusts the conveyance speed of the stretchable laminated sheet L supplied to the laminated sheet stretching mechanism 60. The control device 90 controls the operation of the adjuster 110.

In the manufacturing apparatus 100A of the stretchable laminated sheet L, the conveyance speed of the stretchable laminated sheet L supplied to the laminated sheet stretching mechanism 60 is controlled, and as a result the tension acting on the stretchable laminated sheet L supplied to the laminated sheet stretching mechanism 60 is controlled. For that reason, the occurrence of problems such as excessive tension acting on the stretchable laminated sheet L in the laminated sheet stretching mechanism 60 and damaging the stretchable laminated sheet L or the size of the opening formed in the stretchable laminated sheet L in the laminated sheet stretching mechanism 60 being different from the desired size can be reduced.

(2-2)
In the manufacturing apparatus 100A of the stretchable laminated sheet L, during steady operation for example, the control device 90 controls the operation of the adjuster 110 to make the tension acting on the stretchable laminated sheet L supplied to the laminated sheet stretching mechanism 60 smaller relative to the tension acting on the stretchable laminated sheet L conveyed from the laminating and joining mechanism 48 to the adjuster 110.

In the manufacturing apparatus 100A of the stretchable laminated sheet L, the occurrence of the problem of excessive tension acting on the stretchable laminated sheet L in the laminated sheet stretching mechanism 60 and damaging the stretchable laminated sheet L during steady operation can be reduced.

(2-3)
In the manufacturing apparatus 100A of the stretchable laminated sheet L, when the manufacturing apparatus 100A starts operating or stops operating for example, the control device 90 controls the operation of the adjuster 110 to make the tension acting on the stretchable laminated sheet L supplied to the laminated sheet stretching mechanism 60 greater relative to the tension acting on the stretchable laminated sheet L conveyed from the laminating and joining mechanism 48 to the adjuster 110.

Assuming that the control device 90 does not control the operation of the adjuster 110. In this case, when the manufacturing apparatus 100A of the stretchable laminated sheet L starts operating or stops operating, the tension acting on the stretchable laminated sheet L supplied to the laminated sheet stretching mechanism 60 is smaller compared with that of during steady operation, and there is a possibility that the size of the opening formed in the stretchable laminated sheet L by the laminated sheet stretching mechanism 60 will be smaller compared with that of during steady operation. If the openings are too small, the stretchable laminated sheet L will not have the desired breathability, so the parts of the stretchable laminated sheet L where the openings are too small may not be usable for wearable articles (will be wasted).

In contrast, the manufacturing apparatus 100A of the stretchable laminated sheet L controls the operation of the adjuster 110 to increase the tension acting on the stretchable laminated sheet L supplied to the laminated sheet stretching mechanism 60, so even when the manufacturing apparatus 100A of the stretchable laminated sheet L starts operating or stops operating, openings with an appropriate size can be formed in the stretchable laminated sheet L in the laminated sheet stretching mechanism 60.

(2-4)
In the manufacturing apparatus 100A of the stretchable laminated sheet L, the control device 90 controls the operation of the adjuster 110 to keep the tension acting on the stretchable laminated sheet L supplied to the laminated sheet stretching mechanism 60 constant.

In the manufacturing apparatus 100A of the stretchable laminated sheet L, the tension acting on the stretchable laminated sheet L supplied to the laminated sheet stretching mechanism 60 is constant, so openings with generally the same size tend to always be formed in the stretchable laminated sheet L.

(2-5)
The manufacturing apparatus 100A of the stretchable laminated sheet L includes the guide rollers 120 serving as an example of a first roller. The guide rollers 120 are disposed between the laminating and joining mechanism 48 and the adjuster 110 on the conveyance path and guide the stretchable laminated sheet L that is wound around the guide rollers 120. The adjuster 120 includes the speed adjusting roller 112 serving as an example of a second roller around which the stretchable laminated sheet L is wound. The outer surface of the speed adjusting roller 112 has a higher adhesion or coefficient of friction than the outer surfaces of the guide rollers 120.

In the manufacturing apparatus 100A of the stretchable laminated sheet L, the stretchable laminated sheet L can be gripped by the outer surface of the speed adjusting roller 112 in the adjuster 110, and the conveyance speed of the stretchable laminated sheet L supplied to the laminated sheet stretching mechanism 60 can be adjusted using a single roller.

However, rather than having a configuration using the single speed adjusting roller 112, the adjuster 110 may also use two rollers to sandwich the stretchable laminated sheet L and adjust the conveyance speed of the stretchable laminated sheet L.

(2-6)
The manufacturing apparatus 100A of the stretchable laminated sheet L includes the heater 66 that heats at least one of the pair of gear rolls 62, 64. In the present embodiment, the manufacturing apparatus 100A of the stretchable laminated sheet L includes the heater 66 that heats at least the gear roll 64.

In this manufacturing apparatus 100A of the stretchable laminated sheet L, the stretchable laminated sheet L can be heated and then stretched, so the stretchable laminated sheet L is easily stretched in the laminated sheet stretching mechanism 60.

(2-7)
In the manufacturing apparatus 100A of the stretchable laminated sheet L, the stretchable laminated sheet L is guided between the pair of gear rolls 62, 64 after contacting for a predetermined length the outer peripheral surface of the one of the pair of gear rolls 62, 64 that is heated by the heater 66. In the present embodiment, the stretchable laminated sheet L is guided between the pair of gear rolls 62, 64 after contacting for a predetermined length the outer peripheral surface of the one of the gear roll 64 that is heated by the heater 66.

In the manufacturing apparatus 100A of the stretchable laminated sheet L, the stretchable laminated sheet L is easily sufficiently heated, and the stretchable laminated sheet L is easily sufficiently stretched in the laminated sheet stretching mechanism 60.

(2-8)
The manufacturing apparatus 100A of the stretchable laminated sheet L includes the receiving unit 92 and the control device 90. The receiving unit 92 receives information relating to the size of the opening that the laminated sheet stretching mechanism 60 is to form in the stretchable laminated sheet L. The control device 90 determines the settings of the gear rolls 62, 64 in the laminated sheet stretching mechanism 60 based on the information relating to the size of the opening that the laminated sheet stretching mechanism 60 is to form in the stretchable laminated sheet L.

In the manufacturing apparatus 100A of the stretchable laminated sheet L, the settings of the gear rolls 62, 64 are determined in accordance with the size of the opening to be provided in the stretchable laminated sheet L, so in the laminated sheet stretching mechanism 60, openings with the desired size are easily formed in the stretchable laminated sheet L.

### <Example Modification 1>

The manufacturing apparatus 100A may have the following configuration instead of the configuration "c".

As a method of changing the size of the opening that the laminated sheet stretching mechanism 60 is to form in the stretchable laminated sheet L in accordance with the size of the opening required in the stretchable laminated sheet L, a method is also conceivable where, instead of changing the settings of the gear rolls 62, 64 of the laminated sheet stretching mechanism 60, the control device 90 controls the operation of the adjuster 110 to change the tension acting on the stretchable laminated sheet L supplied to the laminated sheet stretching mechanism 60.

Thus, the control device 90 may determine the control pattern of the adjuster 110 rather than deciding on the settings of the gear rolls 62, 64 in accordance with the size of the opening required in the stretchable laminated sheet L.

An example of a specific configuration that realizes the manufacturing apparatus 100A of example modification 1 will now be described.

The manufacturing apparatus 100A of example modification 1, as in the above embodiment, has a receiving unit 92 that receives information relating to the size of the opening that the laminated sheet stretching mechanism 60 is to form in the stretchable laminated sheet L. Since the receiving unit 92 and the information relating to the size of the opening that the laminated sheet stretching mechanism 60 is to form in the stretchable laminated sheet L have already been described in the second embodiment, description thereof will be omitted here.

The control device 90 of the manufacturing apparatus 100A determines the control pattern of the adjuster 110 based on the information, received by the receiving unit 92, relating to the size of the opening that the laminated sheet stretching mechanism 60 is to form in the stretchable laminated sheet L.

For example, the memories of the control device 90 stores correlation information in which the size of the opening that the laminated sheet stretching mechanism 60 is to form in the stretchable laminated sheet L and control patterns of the adjuster 110 (a method of how to control the rotational speed of the speed regulating motor 114 when the manufacturing apparatus 100A starts operating, when the manufacturing apparatus 100A stops operating, and during steady operation) are correlated with each other. The correlation information here is information based on test results obtained by using a test device for testing the manufacturing apparatus 100A, gear stretching the stretchable laminated sheet L while changing the control pattern of the adjuster 110, and measuring the openings formed by the gear stretching. Furthermore, the correlation information is information relating to correlations between the control pattern of the adjuster 110 and the openings formed by the gear stretching, which is calculated theoretically or obtained by computer simulation.

The control device 90 references the correlation information to determine the control pattern of the adjuster 110 corresponding to the information, received by the receiving unit 92, relating to the size of the opening that the laminated sheet stretching mechanism 60 is to form in the stretchable laminated sheet L.

The manufacturing apparatus 100A may, for example, output the control pattern of the adjuster 110 on which it has decided from the output unit 94 (description regarding the output unit 94 is omitted). Additionally, the operator of the manufacturing apparatus 100A may input the control pattern of the adjuster 110 to the control device 90. Furthermore, the control device 90 may, during the operation of the manufacturing apparatus 100A, control the operation of the adjuster 110 using the settings of the control pattern of the adjuster 110 on which it has determined.

In the manufacturing apparatus 100A of the stretchable laminated sheet L of example modification A, the tension acting on the stretchable laminated sheet L supplied to the laminated sheet stretching mechanism 60 is changed in accordance with the information relating to the size of the opening that the laminated sheet stretching mechanism 60 is to form in the stretchable laminated sheet L.

For that reason, in the laminated sheet stretching mechanism 60, openings with the desired size are easily formed in the stretchable laminated sheet L.

### <Example Modification 2>

In the manufacturing apparatus 100A of the second embodiment, it is assumed that the operator of the manufacturing apparatus 100A manually changes the settings of the gear rolls 62, 64 in accordance with the size of the opening required in the stretchable laminated sheet L.

However, the manufacturing apparatus 100A is not limited to this and may automatically change the settings of the gear rolls 62, 64 in accordance with the size of the opening required in the stretchable laminated sheet L.

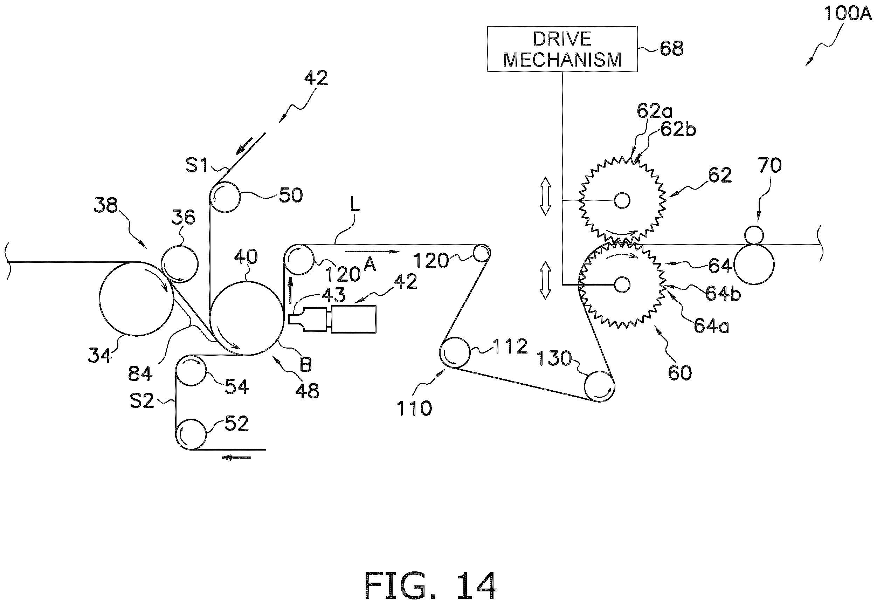

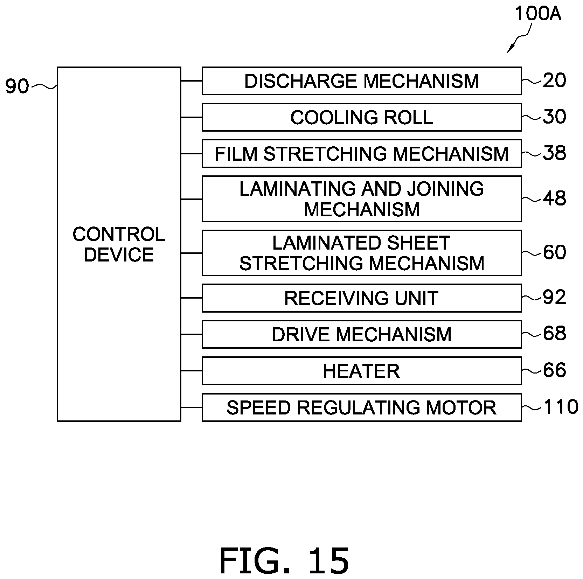

A specific configuration for realizing the manufacturing apparatus 100A of example modification 2 will now be described with reference to FIG. 14 and FIG. 15. FIG. 14 is a schematic view showing the configuration of the manufacturing apparatus 100A of the stretchable laminated sheet L of example modification 2, with illustration of the former of the film F being omitted. FIG. 15 is a control block diagram of the manufacturing apparatus 100A of the stretchable laminated sheet L of example modification 2.

It will be noted that, here, a case where the setting of the gear rolls 62, 64 is the distance setting between the axial center of the gear roll 62 and the axial center of the gear roll 64 will be described as an example.

The manufacturing apparatus 100A of example modification 2 has a drive mechanism 68 that moves at least one of the axial center of the gear roll 62 and the axial center of the gear roll 64 toward and away from that of the other gear roll (see FIG. 14 and FIG. 15). As shown in FIG. 15, the manufacturing apparatus 100A of example modification 2 does not have the output unit 94. However, the manufacturing apparatus 100A is not limited to this and may have the output unit 94.

The manufacturing apparatus 100A of example modification 2 determines, by the same method as that of the manufacturing apparatus 100A of the second embodiment, on the setting of the gear rolls 62, 64 in the laminated sheet stretching mechanism 60 (here, the distance between the axial center of the gear roll 62 and the axial center of the gear roll 64) based on the information, received by the receiving unit 92, relating to the size of the opening that the laminated sheet stretching mechanism 60 is to form in the stretchable laminated sheet L.

Then, the control device 90, instead of outputting from the output unit 94 the settings of the gear rolls 62, 64 on which it has determined, controls the operation of the drive mechanism 68 to adjust the distance between the axial center of the gear roll 62 and the axial center of the gear roll 64 to the distance between the axial center of the gear roll 62 and the axial center of the gear roll 64 on which it has determined.

Finally, the technical thought that can be grasped from the above embodiments is appended below.

A stretchable laminated sheet manufacturing apparatus is an apparatus for manufacturing a stretchable laminated sheet in which a substrate sheet and a stretchable film are disposed on top of each other and in which the substrate sheet and the film are joined together. The stretchable laminated sheet manufacturing apparatus includes a joining unit and a stretcher. The joining unit has a first joining member and a second joining member. The joining unit is configured to sandwich between the first joining member and the second joining member a laminate body in which the substrate sheet and the film that is in an extended state are superimposed and heat seals the substrate sheet and the film to form the stretchable laminated sheet. The stretcher has a pair of gear rolls having recesses and projections in their peripheral surfaces. The stretcher is configured to stretch the stretchable laminated sheet which is conveyed between the pair of gear rolls.

The stretchable laminated sheet manufacturing apparatus of the first aspect can form openings with a sufficient area in and/or around the places where the substrate sheet and the film have been heat sealed by heat sealing the substrate sheet and the film to form the stretchable laminated sheet and then performing stretching with the gear rolls having recesses and projections in their peripheral surfaces. Therefore, it can manufacture the stretchable laminated sheet that has improved breathability.

A stretchable laminated sheet manufacturing apparatus of a second aspect is the stretchable laminated sheet manufacturing apparatus of the first aspect, including a former and a film stretcher. The former is configured to form the film from a raw material. The film stretcher is disposed between the former and the joining unit in a conveyance direction of the film and stretches the film. An extension ratio of the film conveyed between the film stretcher and the joining unit to the film at its natural length is greater than an extension ratio of the film conveyed between the former and the film stretcher to the film at its natural length. An extension ratio of the film of the stretchable laminated sheet in the stretcher to the film at its natural length is greater than the extension ratio of the film conveyed between the film stretcher and the joining unit to the film at its natural length.

**In** the stretchable laminated sheet manufacturing apparatus of the second aspect, the thickness of the film can be reduced by extending the film in stages. **In** particular, in this stretchable laminated sheet manufacturing apparatus, by using the film to form the stretchable laminated sheet and thereafter further stretching the film, the thickness of the film can be reduced while reducing damage to the film.

A stretchable laminated sheet manufacturing apparatus of a third aspect is the stretchable laminated sheet manufacturing apparatus of the first aspect or the second aspect, further including an adjuster and a speed controller. The adjuster is disposed between the joining unit and the stretcher on a conveyance path of the stretchable laminated sheet and is configured to adjust the conveyance speed of the stretchable laminated sheet supplied to the stretcher. The speed controller is configured to control the operation of the adjuster.

In the stretchable laminated sheet manufacturing apparatus of the third aspect, the conveyance speed of the stretchable laminated sheet supplied to the stretcher is controlled, and as a result the tension acting on the stretchable laminated sheet supplied to the stretcher is controlled. For that reason, the occurrence of problems such as excessive tension acting on the stretchable laminated sheet in the stretcher and damaging the stretchable laminated sheet or the size of the opening formed in the stretchable laminated sheet in the stretcher being different from the desired size can be reduced.

A stretchable laminated sheet manufacturing apparatus of a fourth aspect is the stretchable laminated sheet manufacturing apparatus of the third aspect, wherein the speed controller is configured to control, during steady operation, the operation of the adjuster to make a tension acting on the stretchable laminated sheet supplied to the stretcher smaller relative to a tension acting on the stretchable laminated sheet conveyed from the joining unit to the adjuster.

In the stretchable laminated sheet manufacturing apparatus of the fourth aspect, the occurrence of the problem of excessive tension acting on the stretchable laminated sheet in the stretcher and damaging the stretchable laminated sheet during steady operation can be reduced.

A stretchable laminated sheet manufacturing apparatus of a fifth aspect is the stretchable laminated sheet manufacturing apparatus of the third aspect or the fourth aspect, wherein when the manufacturing apparatus starts operating or stops operating, the speed controller is configured to control the operation of the adjuster to make a tension acting on the stretchable laminated sheet supplied to the stretcher greater relative to a tension acting on the stretchable laminated sheet conveyed from the joining unit to the adjuster.

Assuming that the speed controller does not control the operation of the adjuster. In thi case, when the stretchable laminated sheet manufacturing apparatus starts operating or stops operating, the tension acting on the stretchable laminated sheet supplied to the stretcher becomes smaller compared with that of during steady operation, and there is a possibility that the size of the opening formed in the stretchable laminated sheet by the stretcher will be smaller compared with that of during steady operation. If the openings are too small, the stretchable laminated sheet will not have the desired breathability, so the parts of the stretchable laminated sheet where the openings are too small may not be usable for wearable articles (will be wasted).

In contrast, the stretchable laminated sheet manufacturing apparatus of the fifth aspect controls the operation of the adjuster to increase the tension acting on the stretchable laminated sheet supplied to the stretcher, so even when the stretchable laminated sheet manufacturing apparatus starts operating or stops operating, openings with an appropriate size can be formed in the stretchable laminated sheet in the stretcher.

A stretchable laminated sheet manufacturing apparatus of a sixth aspect is the stretchable laminated sheet manufacturing apparatus of the third aspect, wherein the speed controller is configured to control the operation of the adjuster to keep a tension acting on the stretchable laminated sheet supplied to the stretcher constant.

In the stretchable laminated sheet manufacturing apparatus of the sixth aspect, the tension acting on the stretchable laminated sheet supplied to the stretcher is constant, so openings with generally the same size tend to always be formed in the stretchable laminated sheet.

A stretchable laminated sheet manufacturing apparatus of a seventh aspect is the stretchable laminated sheet manufacturing apparatus of any of the third aspect to the sixth aspect, further includes a first roller. The first roller is disposed between the joining unit and the adjuster on the conveyance path and is configured to guide the stretchable laminated sheet that is wound around it. The adjuster includes a second roller around which the stretchable laminated sheet is wound. An outer surface of the second roller has a higher adhesion or coefficient of friction than an outer surface of the first roller.

In the stretchable laminated sheet manufacturing apparatus of the seventh aspect, the stretchable laminated sheet can be gripped by the outer surface of the second roller in the adjuster, and the conveyance speed of the stretchable laminated sheet supplied to the stretcher can be adjusted using a single roller.

A stretchable laminated sheet manufacturing apparatus of an eighth aspect is the stretchable laminated sheet manufacturing apparatus of any of the first aspect to the seventh aspect, further including a heater configured to heat at least one of the pair of gear rolls.

In the stretchable laminated sheet manufacturing apparatus of the eighth aspect, the stretchable laminated sheet can be heated and then stretched, so the stretchable laminated sheet is easily stretched in the stretcher.

A stretchable laminated sheet manufacturing apparatus of a ninth aspect is the stretchable laminated sheet manufacturing apparatus of the eighth aspect, wherein the stretchable laminated sheet is configured to be guided between the pair of gear rolls after contacting for a predetermined length an outer peripheral surface of the one of the pair of gear rolls that is heated by the heater.

In the stretchable laminated sheet manufacturing apparatus of the ninth aspect, the stretchable laminated sheet is easily sufficiently heated, and the stretchable laminated sheet is easily sufficiently stretched in the stretcher.

A stretchable laminated sheet manufacturing apparatus of a tenth aspect is the stretchable laminated sheet manufacturing apparatus of any of the first aspect to the ninth aspect, further including a receiving unit and a determining unit. The receiving unit is configured to receive information relating to a size of an opening that the stretcher is to form in the stretchable laminated sheet. The determining unit is configured to determine a setting of the gear rolls in the stretcher based on the information relating to the size of openings that the stretcher is to form in the stretchable laminated sheet.

In the stretchable laminated sheet manufacturing apparatus of the tenth aspect, the settings of the gear rolls are decided on in accordance with the size of the opening to be provided in the stretchable laminated sheet, so in the stretcher, openings with the desired size are easily formed in the stretchable laminated sheet.

A stretchable laminated sheet manufacturing apparatus of an eleventh aspect is the stretchable laminated sheet manufacturing apparatus of any of the first aspect to the ninth aspect, further comprising a receiving unit. The receiving unit is configured to receive information relating to a size of an opening that the stretcher is to form in the stretchable laminated sheet. The tension acting on the stretchable laminated sheet supplied to the stretcher is changed in accordance with the information relating to the size of the opening that the stretcher is to form in the stretchable laminated sheet.

In the stretchable laminated sheet manufacturing apparatus of the eleventh aspect, the tension in the stretchable laminated sheet supplied to the stretcher is changed in accordance with the size of the opening to be provided in the stretchable laminated sheet, so in the stretcher, openings with the desired size are easily formed in the stretchable laminated sheet.

A stretchable laminated sheet manufacturing method of a twelfth aspect is a method of manufacturing a stretchable laminated sheet in which a substrate sheet and a stretchable film are disposed on top of each other and in which a pair of the substrate sheets and the film are joined together. The stretchable laminated sheet manufacturing method includes a sealing step and a stretching step. In the sealing step, the substrate sheet and the film that is in an extended state are heat sealed to form the stretchable laminated sheet. In the stretching step, the stretchable laminated sheet which is conveyed between a pair of gear rolls having recesses and projections in their peripheral surfaces is stretched by the pair of gear rolls.

The stretchable laminated sheet manufacturing method of the twelfth aspect can form openings with a sufficient area in and/or around the places where the substrate sheet and the film have been heat sealed (see FIG. 9 and FIG. 11) by heat sealing the substrate sheet and the film to form the stretchable laminated sheet and then performing stretching with the gear rolls having recesses and projections in their peripheral surfaces. Therefore, it is possible to manufacture the stretchable laminated sheet that has improved breathability.

A wearable article of a thirteenth aspect comprises a waist member and an absorbent body. The waist member is disposed around a waist of a wearer when the wearable article is worn. The absorbent body is joined to the waist member and disposed in a crotch region of the wearer. The waist member includes a waistline portion that is disposed on an upper end of the waist member and disposed around a waistline of the wearer when the wearable article is worn. A stretchable laminated sheet manufactured by the manufacturing method of the twelfth aspect is used for the waistline portion.

A wearable article of a fourteenth aspect includes a waist member and an absorbent body. The waist member is disposed around a waist of a wearer when the wearable article is worn. The absorbent body is joined to the waist member and disposed in a crotch region of the wearer when the wearable article is worn. The waist member includes a waistline portion and a lower waist portion. The waistline portion is configured to be disposed on an upper end of the waist member and is configured to be disposed around a waistline of the wearer when the wearable article is worn. The lower waist portion is configured to be disposed below the waistline stretchable portion when the wearable article is worn. A stretchable laminated sheet manufactured by the manufacturing method of the twelfth aspect is used for the lower waist portion.

A wearable article of a fifteenth aspect includes a waist member, an absorbent body, and a connecting member. The waist member is configured to be disposed around a waist of a wearer when the wearable article is worn. The absorbent body is joined to the waist member and is configured to be disposed in a crotch region of the wearer when the wearable article is worn. The connecting member is attached to a connecting member attachment portion provided on at least one of left and right end portions of the waist member. The connecting member is configured to be connected to a connected portion of the waist member when the wearable article is worn. A stretchable laminated sheet manufactured by the manufacturing method of the twelfth aspect is used for the connecting member attachment portion or the connected portion.

A wearable article of a sixteenth aspect includes a waist member, an absorbent body, and a connecting member. The waist member is disposed around a waist of a wearer when the wearable article is worn. The absorbent body is joined to the waist member and is configured to be disposed in the crotch region of a wearer when the wearable article is worn. The connecting members are attached to connecting member attachment portions provided on at least one of left and right end portions of the waist member. The connecting member is configured to be connected to a connected portion of the waist member when the wearable article is worn. A stretchable laminated sheet manufactured by the manufacturing method of the twelfth aspect is used for the connecting member.

In the wearable articles of the thirteenth aspect to the sixteenth aspect, wearable articles that have improved breathability are realized because of the stretchable laminated sheet.

A wearable article of a seventeenth aspect includes a waist member, an absorbent body, and a connecting member. The waist member is configured to be disposed around a waist of a wearer when the wearable article is worn. The absorbent body is joined to the waist member and is configured to be disposed in the crotch region of a wearer when the wearable article is worn. The connecting member is attached to a connecting member attachment portion provided on at least one of left and right end portions of the waist member. The connecting member is configured to be connected to a connected portion of the waist member when the wearable article is worn. A stretchable laminated sheet, in which a substrate sheet and a stretchable film are joined together in a superimposed state and in which openings are formed in joints between the substrate sheet and the film, is used for at least one of the waist member and the connecting member.

In the wearable article of the seventeenth aspect, the stretchable laminated sheet, in which openings are also formed in the joints between the substrate sheet and the film, is used, so the wearable article that has improved breathability is realized.

### REFERENCE SIGNS LIST

- 20: Discharge Mechanism (Former)
- 30: Cooling Roll (Former)
- 38: Film Stretching Mechanism (Film Stretcher)
- 48: Laminating and Joining Mechanism (Joining Unit)
- 60: Laminated Sheet Stretching Mechanism (Stretcher)
- 62, 64: Gear Rolls
- 66: Heater
- 68: Axial Drive Mechanism
- 90: Control Device (Speed Controller, Determining unit)
- 92: Receiving Unit
- 94: Output Unit
- 100, 100A: Stretchable Laminated Sheet Manufacturing Apparatus
- 110: Adjuster
- 112: Speed Adjusting Roller (Second Roller)
- 120: Guide Roller (First Roller)
- 300: Wearable Article
- 310: Waist Member
- 312: Waistline Portion
- 314: Lower Waist Portion
- 320: Absorbent body
- 400: Wearable Article
- 410: Front Waist Portion (Waist Member)
- 412: Waistline Portion
- 414: Lower Waist Portion
- 416: Attachment Portion (Connecting Member Attachment Portion)
- 420: Back Waist Portion (Waist Member)
- 422: Waistline Portion
- 424: Lower Waist Portion
- 426: Loop Portion (Connected Portion)
- 430: Crotch Portion (Absorbent Body)
- 440: Absorbent Article (Absorbent Body)
- 450: Fastening Tape (Connecting Member)
- B: Laminate Body
- F: Film
- L: Stretchable Laminated Sheet
- S1: First Nonwoven Fabric Sheet (Substrate Sheet)
- S2: Second Nonwoven Fabric Sheet (Substrate Sheet)

## Claims

1. An apparatus for manufacturing a stretchable laminated sheet in which a substrate sheet and a stretchable film are disposed on top of each other and in which the substrate sheet and the film are joined together, the stretchable laminated sheet manufacturing apparatus comprising:
a joining unit having a first joining member and a second joining member configured to sandwich therebetween a laminate body in which the substrate sheet and the film that is in an extended state are superimposed, and heat seal the substrate sheet and the film to form the stretchable laminated sheet; and
a stretcher having a pair of gear rolls having recesses and projections in their peripheral surfaces and configured to stretch the stretchable laminated sheet which is conveyed between the pair of gear rolls.

2. The stretchable laminated sheet manufacturing apparatus according to claim 1, further comprising
a former configured to form the film from a raw material and
a film stretcher disposed between the former and the joining unit in a conveyance direction of the film and stretches the film,
wherein
an extension ratio of the film conveyed between the film stretcher and the joining unit to the film at its natural length is greater than an extension ratio of the film conveyed between the former and the film stretcher to the film at the natural length, and
an extension ratio of the film of the stretchable laminated sheet in the stretcher to the film at the natural length is greater than the extension ratio of the film conveyed between the film stretcher and the joining unit to the film at its natural length.

3. The stretchable laminated sheet manufacturing apparatus according to claim 1 or 2, further comprising
an adjuster that is disposed between the joining unit and the stretcher on a conveyance path of the stretchable laminated sheet and configured to adjust the conveyance speed of the stretchable laminated sheet supplied to the stretcher and
a speed controller configured to control the operation of the adjuster.

4. The stretchable laminated sheet manufacturing apparatus according to claim 3, wherein the speed controller is configured to control, during steady operation, the operation of the adjuster to make a tension acting on the stretchable laminated sheet supplied to the stretcher smaller relative to a tension acting on the stretchable laminated sheet conveyed from the joining unit to the adjuster.

5. The stretchable laminated sheet manufacturing apparatus according to claim 3 or 4, wherein the speed controller is configured to control, when the manufacturing apparatus starts operating or stops operating, the operation of the adjuster to make a tension acting on the stretchable laminated sheet supplied to the stretcher greater relative to a tension acting on the stretchable laminated sheet conveyed from the joining unit to the adjuster.

6. The stretchable laminated sheet manufacturing apparatus according to claim 3, wherein the speed controller is configured to control the operation of the adjuster to keep a tension acting on the stretchable laminated sheet supplied to the stretcher constant.

7. The stretchable laminated sheet manufacturing apparatus according to any one of claims 3 to 6, further comprising a first roller disposed between the joining unit and the adjuster on the conveyance path and configured to guide the stretchable laminated sheet that is wound around it,
wherein
the adjuster includes a second roller around which the stretchable laminated sheet is configured to wound, and
an outer surface of the second roller has a higher adhesion or coefficient of friction than an outer surface of the first roller.

8. The stretchable laminated sheet manufacturing apparatus according to any one of claims 1 to 7, further comprising a heater configured to heat at least one of the pair of gear rolls.

9. The stretchable laminated sheet manufacturing apparatus according to claim 8, wherein the stretchable laminated sheet is configured to be guided between the pair of gear rolls after contacting for a predetermined length an outer peripheral surface of the one of the pair of gear rolls that is heated by the heater.

10. The stretchable laminated sheet manufacturing apparatus according to any one of claims 1 to 9, further comprising
a receiving unit configured to receive information relating to a size of an opening that the stretcher is to form in the stretchable laminated sheet, and
a determining unit configured to determine on a setting of the gear rolls in the stretcher based on the information.

11. The stretchable laminated sheet manufacturing apparatus according to any one of claims 1 to 9, further comprising a receiving unit configured to receive information relating to a size of an opening that the stretcher is to form in the stretchable laminated sheet,
wherein
a tension acting on the stretchable laminated sheet supplied to the stretcher is changed in accordance with the information.

12. A method of manufacturing a stretchable laminated sheet in which a substrate sheet and a stretchable film are disposed on top of each other and in which the substrate sheet and the film are joined together, the stretchable laminated sheet manufacturing method comprising:
a sealing step of heat sealing the substrate sheet and the film that is in an extended state to form the stretchable laminated sheet; and
a stretching step of stretching, with a pair of gear rolls having recesses and projections in their peripheral surfaces, the stretchable laminated sheet which is conveyed between the pair of gear rolls.

13. A wearable article comprising:
a waist member configured to be disposed around a waist of a wearer; and
an absorbent body joined to the waist member and configured to be disposed in a crotch region of the wearer;
wherein
the waist member includes a waistline portion configured to be disposed on an upper end of the waist member and to be disposed around a waistline of the wearer when the wearable article is worn, and
a stretchable laminated sheet manufactured by the manufacturing method of claim 12 is used for the waistline portion.

14. A wearable article comprising:
a waist member configured to be disposed around a waist of a wearer; and
an absorbent body joined to the waist member and configured to be disposed in a crotch region of the wearer;
wherein
the waist member includes a waistline portion configured to be disposed on an upper end of the waist member and to be disposed around a waistline of the wearer and a lower waist portion configured to be disposed below the waistline portion when the wearable article is worn, and
a stretchable laminated sheet manufactured by the manufacturing method of claim 12 is used for the lower waist portion.

15. A wearable article comprising:
a waist member configured to be disposed around a waist of a wearer;
an absorbent body joined to the waist member and configured to be disposed in a crotch region of the wearer; and
a connecting member attached to a connecting member attachment portion provided on at least one of left and right end portions of the waist member and configured to be connected to a connected portion of the waist member when the wearable article is worn,
wherein
a stretchable laminated sheet manufactured by the manufacturing method of claim 12 is used for the connecting member attachment portion or the connected portion.

16. A wearable article comprising:
a waist member configured to be disposed around a waist of a wearer;
an absorbent body joined to the waist member and configured to be disposed in a crotch region of the wearer; and
a connecting member attached to a connecting member attachment portion provided on at least one of left and right end portions of the waist member and configured to be connected to a connected portion of the waist member when the wearable article is worn,
wherein
a stretchable laminated sheet manufactured by the manufacturing method of claim 12 is used for the connecting member.

17. A wearable article comprising:
a waist member configured to be disposed around a waist of a wearer;
an absorbent body that is joined to the waist member and disposed in a crotch region of a wearer; and
a connecting member attached to a connecting member attachment portion provided on at least one of left and right end portions of the waist member and configured to be connected to a connected portion of the waist member when the wearable article is worn,
wherein
a stretchable laminated sheet, in which a substrate sheet and a stretchable film are joined together in a superimposed state and in which openings are formed in joints between the substrate sheet and the film, is used for at least one of the waist member and the connecting member.
